# EUROPEAN PATENT SPECIFICATION

(11) **EP 2 681 233 B1**
(45) Date of publication and mention of the grant of the patent: **24.10.2018**
(21) Application number: 12704427.9
(22) Date of filing: 20.02.2012
(51) Int. Cl.: C07K 14/415, C12N 15/82, A01H 5/00

(54) **POWDERY MILDEW RESISTANCE PROVIDING GENES IN CUCUMIS SATIVUS**
RESISTENZ GEGENÜBER ECHTEM MEHLTAU VERLEIHENDE GENE IN CUCUMIS SATIVUS
GÈNES DU CUCUMIS SAVITUS CONFÉRANT UNE RÉSISTANCE À L'OÏDIUM

(30) Priority: 01.03.2011 WO PCT/EP2011/053054
(43) Date of publication of application: 08.01.2014
(73) Proprietor: Enza Zaden Beheer B.V., 1602 DB Enkhuizen (NL); KEYGENE N.V., 6708 PW Wageningen (NL)
(72) Inventor: DIERGAARDE, Paul Johan, NL-3823 EA Amersfoort (NL); VAN ENCKEVORT, Leonora Johanna Gertruda, NL-6707 GJ Wageningen (NL); POSTHUMA, Karin Ingeborg, NL-1601 AJ Enkhuizen (NL); PRINS, Marinus Willem, NL-3818 VD Amersfoort (NL)
(74) Representative: van Kooij, Adriaan
(86) International application number: PCT/EP2012/052843
(87) International publication number: WO 2013/017293

(56) References cited:
- WO-A1-2008/017706
- WO-A2-98/04586
- RALPH PANSTRUGA: "Discovery of Novel Conserved Peptide Domains by Ortholog Comparison within Plant Multi-Protein Families", PLANT MOLECULAR BIOLOGY, KLUWER ACADEMIC PUBLISHERS, DORDRECHT, NL, vol. 59, no. 3, 1 October 2005 (2005-10-01), pages 485-500, XP019262780, ISSN: 1573-5028, DOI: 10.1007/S11103-005-0353-0 & DATABASE EMBL [Online] 13 March 2005 (2005-03-13), "Capsicum annuum MLO1 (MLO1) mRNA, complete cds.", retrieved from EBI accession no. EM_PL:AY934528 Database accession no. AY934528 & DATABASE UniProt [Online] 12 April 2005 (2005-04-12), "SubName: Full=MLO1;", retrieved from EBI accession no. UNIPROT:Q5BMD0 Database accession no. Q5BMD0
- HONG CHENG ET AL: "Molecular cloning and expression analysis of CmMlo1 in melon", MOLECULAR BIOLOGY REPORTS, vol. 39, no. 2, 1 February 2012 (2012-02-01), pages 1903-1907, XP055024430, ISSN: 0301-4851, DOI: 10.1007/s11033-011-0936-6 & DATABASE EMBL [Online] 27 October 2011 (2011-10-27), "Cucumis melo Mlo1 mRNA, complete cds.", retrieved from EBI accession no. EM_PL:FJ713541 Database accession no. FJ713541 & DATABASE UniProt [Online] 25 January 2012 (2012-01-25), "SubName: Full=Mlo1;", retrieved from EBI accession no. UNIPROT:G7Z0K6 Database accession no. G7Z0K6
- SCHWEIZER P ET AL: "Double-stranded RNA interferes with gene function at the single-cell level in cereals", THE PLANT JOURNAL, BLACKWELL SCIENTIFIC PUBLICATIONS, OXFORD, GB, vol. 24, no. 6, 1 December 2000 (2000-12-01), pages 895-903, XP002397482, ISSN: 0960-7412
- PANSTRUGA R: "Serpentine plant MLO proteins as entry portals for powdery mildew fungi", BIOCHEMICAL SOCIETY TRANSACTIONS, vol. 33, April 2005 (2005-04), pages 389-392, XP009158493, ISSN: 0300-5127

## Description

The present invention relates to plants comprising a powdery mildew resistance providing gene and seeds, embryos or other propagation material thereof.

Powdery mildew (PM) is one of the main fungal diseases known in plants belonging to the *Cucumis* family such as *Cucumis sativus* (cucumber), both in the field and greenhouse.

Powdery mildew diseases are generally caused by many different species of fungi of the order *Erysiphales.* The disease is characterized by distinctive symptoms such as white powder-like spots on the leaves and stems. Generally, the lower leaves are the most affected, but the mildew can appear on any part of the plant that is exposed above ground. As the disease progresses, the spots get larger and thicker as massive numbers of spores form, and the mildew spreads up and down the length of the plant such on the stem and even the fruits.

Severely affected leaves can become dry and brittle, or can wither and die. Because of the infection, the fruits can be smaller in size, fewer in number, less able to be successfully stored, sun scalded, incompletely ripe, and having a poor flavor. It may also predispose plants to be more vulnerable to other pathogens. Eventually, the plant can die.

Powdery mildew can, amongst others, be caused by the fungus *Sphaerotheca fuliginea* (recently renamed: *Podosphaera xanthii* also designated as *Oidium erysiphoides)* and/or *Erysiphe cichoracearum DC* (recently renamed: *Golovinomyces cichoracearum* also designated *as Oidium chrysanthemi*).

Panstruga, 2005, Plant Mol. Biol. 59: 485-500, discloses novel conserved peptide domains by otholog comparison within plant multi-protein families using sequence allignments of heptahelical Arabidopsis MLO proteins.

Panstruga, 2005, Biochem. Soc. Trans. 33: 389-393, discloses serpentine plant MLO proteins as entry portals for powdery mildew fungi.

Considering the economic importance of Cucumis plant species, such as cucumber, there is a continued need in the art to provide powdery mildew resistance providing genes.

In view of the above need in the art, it is an object of the present invention, amongst other objects, to meet this need.

According to the present invention, this object, amongst other objects, is met by an powdery mildew resistance conferring gene comprising *Cucumis sativus* plant as defined in appended claim 1.

Specifically, this object of the present invention, amongst other objects, is met by a powdery mildew resistance conferring gene comprising *Cucumis sativus* plant, wherein the amino acid sequence encoded by said resistance conferring gene is SEQ ID No. 2 and wherein said resistance conferring gene is impaired.

Impaired resistance conferring gene is meant to indicate a gene providing a reduced, or even absent, susceptibility to powdery mildew caused by fungi indicated by powder-like spots on the leaves and stems , such as fungi belonging to the order *Erysiphales* such as *Sphaerotheca fuliginea* (recently renamed: *Podosphaera xanthii* also designated as *Oidium erysiphoides)* and/or *Erysiphe cichoracearum DC.*

The impaired resistance conferring gene is a mutated gene. The mutation of the gene can through different mechanisms results in impairment. For example, mutations in protein encoding DNA sequences may lead to mutated, truncated or non-functional proteins. Mutations in noncoding DNA sequences may cause alterantive splicing, translation or protein trafficing. Alternatively, a mutation resulting in an altered transcriptional activity of a gene, which determines the amount of mRNA available for translation to protein, may results in low levels, or absence, of proteins. Additionally, the impairment of gene function may be caused after translation, i.e. at protein level.

Impairment according to the present invention is also indicated by observing a powdery mildew resistance in a *Cucumis sativus* plant comprising a gene which as mutated at the protein level as compared to SEQ ID No. 2 provided herein or no expression of SEQ ID No. 2 provided herein is observed.

Impaired is also indicated herein as a non-functional protein. Although the function of the present genes is not yet identified, a non-functional protein can be readily determined by establishing powdery mildew resistance (non-functional) or powdery mildew susceptibility (functional) in a plant. A powdery mildew resistance (non-functional) plant is indicated by comprising a gene which as mutated at the protein level as compared to SEQ ID No. 2 provided herein or no expression of SEQ ID No. 2 provided herein is observed.

Functional and non-functional proteins can also be determined using complementation experiments. For example, transforming a resisitant powdery mildew *Cucumis sativus* plant with the disclosed gene or proteins will result in a powdery mildew susceptible *Cucumis sativus* plant when the gene or protein is functional while the *Cucumis sativus* plant will remain resistant when the gene or protein is non-functional.

According to the present invention, in the present powdery mildew resistance conferring gene comprising *Cucumis sativus* plant powdery mildew resistance is provided when the gene is impaired. Impaired can be indicated by the absence, or decrease of a functional, or non-muted, protein identified herein as SEQ ID No. 2. In the art, many mechanisms are known resulting in the impairment of a gene either at the transcription, translation or protein level.

For example, impairment at the transcription level can be the result of one or more mutations in transcription regulation sequences, such as promoters, enhancers, and initiation, termination or intron splicing sequences. These sequences are generally located 5' of, 3' of, or within the coding sequence represented by SEQ ID No. 1. Impairment can also be provided by a deletion, rearrangement or insertion in the present genes.

Impairment at the translation level can be provided by a premature stop-codons or other RNA -> protein controlling mechanisms (such as splicing) or posttranslational modifications influencing, for example, protein folding or cellular trafficking.

Impairment at the protein level can be provided by truncated, misfolded or disturbed protein-protein interactions.

Independent of the underlying mechanism, impairment according to the present invention is indicated by an decrease or absence a functional protein according to SEQ ID No. 2.

Impairment according to the present invention is provided by one or more mutations in the present genes resulting in the absence of a protein expression product. As indicated, these mutations can cause a defective expression at the transcription or translation level.

Impairment according to the present invention is alsom provided by one or more mutations in the present gene resulting in a non-functional protein expression product. A non-functional protein expression product can, for example, be caused by premature stop-codons, incorrect translation or post-translational processing or by insertions, deletions or amino acid changes .

Disclosed, using molecular biology methods, is impairment accomplished by gene silencing, for example using siRNA or knocking out. Methods based on EMS or other mutagenic chemical compounds capable of randomly change nucleic acids into other nucleotides are also contemplated. Detection of such mutations typically involves high sensitivity melting curve analyses or nucleotide sequencing-based TILLING procedures.

Disclosed are nucleotide and amino acid sequences with more than 70%, preferably more than 80%, more preferably more than 90% and most preferably more than 95% sequence identity either at the nucleotide level or the amino acid level.

Sequence identity as used herein is defined as the number of identical consequtive aligned nucleotides, or amino acids, over the full length of the present sequences divided by the number of nucleotides, or amino acids, of the full length of the present sequences and multiplied by 100%.

For example, a sequence with 80% identity to SEQ ID No. 1 comprises over the total length of 1782 nucleotides of SEQ ID No. 15 1426 identical aligned consequtive nucleotides, i.e., 1426/1782 * 100% = 80%.

The present invention relates to Cucumis sativus plants comprising in their genome an impaired powdery mildew resistance conferring gene, i.e., plants not expressing a functional protein of SEQ ID No. 2.

In general, and preferably, the present plants will be homozygous for the present impaired gene, i.e., comprising two impaired powdery mildew resistance conferring genes, wherein the cDNA sequence transcribed from said resistance conferring gene is SEQ ID No. 1.

Considering the benefits of the present plants, i.e., providing powdery mildew resistance in cucumber plants, the invention also relates to seeds, plant parts or propagation material capable of providing the present powdery mildew resistant cucumber plants which seeds, plant parts or propagation material comprise an impaired powdery mildew resistance conferring gene, wherein the cDNA sequence transcribed from said resistance conferring gene is SEQ ID No. 1.

Disclosed is the use of the present powdery mildew resistance conferring gene or the present isolated amino acid sequence for providing a powdery mildew resistant cucumber plants (*Cucumis sativus*)*.* As indicated, the present use is based on impairment, either at the expression or protein level, of the gene described herein and can be readily determined by the presently provided cDNA and amino acid sequences optionally in combination with determination of the presence or absence of powdery mildew resistance and/or in combination with complementation assays.

The present invention will be further described in the examples below. In the example, reference is made to figures wherein:
- **Figure 1:**: shows Relative expression levels of CsKIP2 in a selection of cucumber germplasm. Average values of expression per group either in absence (-) or presence (+) of a transposon are added including standard deviation error bars. Bar colors indicate the reference gene used to make the calculations.
- **Figure 2:**: shows DNA fragments of CsKIP2 amplified with primers ID3 and ID4 and visualized by gel electrophoresis (2% agarose, 10v/cm, 40 minutes)
- **Figure 3:**: shows cDNA sequence alignment of lines with absence (top strand) and presence (bottom strand) of a transposon-like elemenent in CsKIP2 genomic DNA. Here, a 72 bp deletion is visible in cDNA derived from lines with the transposon-like element present. Primer binding positions are shown in bold italic characters
- **Figure 4:**: shows an amino acid alignment of CsKIP9 alleles of powdery mildew susceptible and powdery mildew resistant CsKIP9 alleles.

### Examples

### Example 1: Cucumber germplasm screen for contribution of CsKIP2 expression levels and allelic variants to resistance/susceptibility

### Introduction

Impairment of functioning of genes can be caused by different mechanisms. Mutations in protein encoding DNA sequences may be causal for loss-of-function alleles or genes with a change in characteristics. Alternatively, altered transcriptional activity of a gene, which determines the amount of mRNA available for translation to protein, may results in low levels of available proteins. Additionally, the impairment of gene function may be caused after translation, i.e. at protein level.

The present examples shows that a mutation (deletion) in the coding sequence of *CsKIP2* provides powdery mildew resistance.

### Material and Methods

A total of twelve Cucumber germplasm lines varying in powdery mildew resistance levels were selected for analysis. Seeds were germinated under standard greenhouse conditions. Hypocotyls were infected with a local powdery mildew isolate 7 days past sowing followed by infection of the first true leaf 14 days past sowing. Evaluation of reaction phenotypes was performed 28 days past sowing (21 and 14 days post infection for hypocotyls) and phenotypes are scored on a scale of 1-9, where 1 is fully susceptible and 9 is fully resistant.

Material of infected plants was collected for subsequent RNA isolation according to standard procedures (Machery-Nagel RNA Plant). RNA isolation was followed by cDNA synthesis using a standard Oligo-dT primer combined with a reverse transcriptase (Finnzymes) with 1 µg total RNA input.

Expression levels of *CsKIP2* were determined with *CsKIP2* specific PCR primer pair (**table 1,** ID1 ID2) amplifying a DNA fragment from exon 5 to exon 7 with a size specific to cDNA, highly different of the product size derived from genomic DNA. In addition, control fragments functioning as internal reference were amplified from three different housekeeping genes i.e. Elongation Factor 1-alpha (EF-1, A. thaliana ortholog Atlg07920.1), Protein Phosphatase 2a sununit a2 (PDF2, A. thaliana ortholog At3g25800.1) and Helicase domaing containing protein 1 (HEL1, A. thaliana ortholog At1g58050.1).

For specific real time detection of dsDNA during PCR amplification, LCGreen (Idaho Technologies) was added to the PCR reaction mixture at 0.5 x concentration. Calculations were made using the ΔΔCt method.

For the detection of allelic variants with *CsKIP2,* a specific region was targeted in the cDNA. This region is suspected to house a transposon-like element (exon 11 transposon). Primers **(table 1,** ID3 ID4)) designed to specifically amplify exon 9 (partial), 10 and 11 (partial) of *CsKIP2* were used for the detection of this fragment.

**Table 1: CsKIP2 specific PCR primers**

| | |
|---|---|
| ID1: | 5'CGACACTTGAGCTTCTGGAG 3' |
| ID2: | 5'GCAAGATGTGCAACAATGAATC 3' |
| ID3: | 5'CCCGCAATGTGGCTATTTGCTGT 3' |
| ID4: | 5'CCCGAGGCTGAACGACCGGA 3' |

### Results

A selection of 12 germplasm lines from the powdery mildew disease test was made for subsequent expression studies and detection of allelic variation of *CsKIP2.* Presence or absence of a transposon-like element suspected to be the causative factor of powdery mildew resistance in genomic DNA was done before starting expression studies in order to investigate its effect on expression.

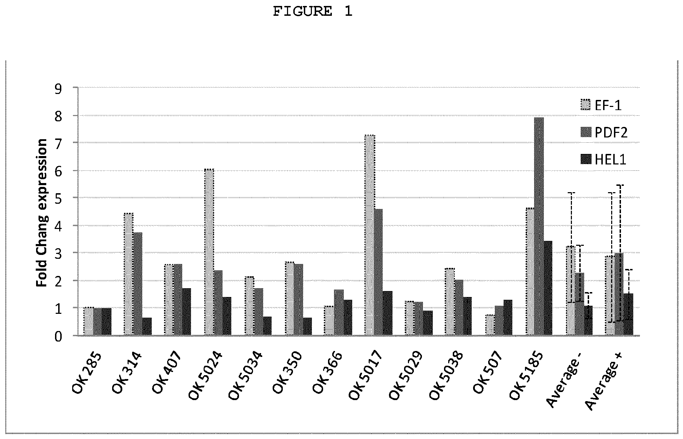

Expression of *CsKIP2* in leaf material derived from the selected plants was determined based on the control genes. The results show no general effect of expression based on the obtained data. On average, expression levels observed are similar (**Figure 1**)

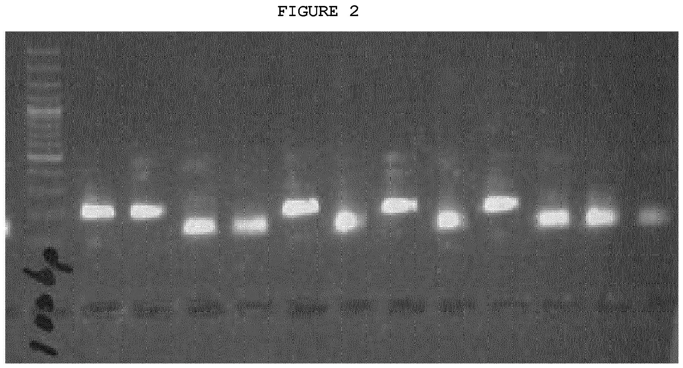

After determination of expression levels, the allelic variation in the transposon-like element was investigated. The fragment amplified with primers ID3 and ID4 produced a variable fragment of size 199 bp or 127 bp (**Figure 2**). The smaller fragment was found strictly in resistant plants and correlates to presence of the transposon in genomic DNA.

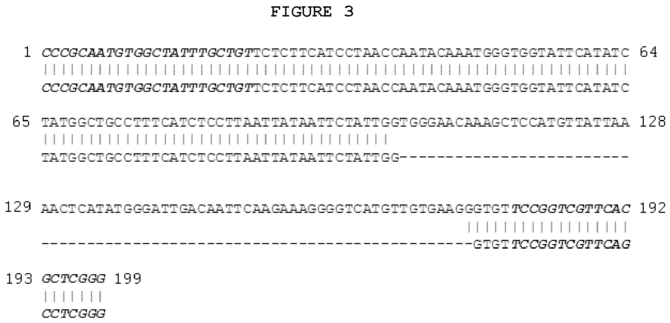

The sequence of the fragments was found to be highly similar except for a 72 bp deletion in exon 11, centered around the original position of the transposon in genomic DNA (**Figure 3**).

### Conclusions

Expression analysis of *CsKIP2* in leaf material from infected cucumber plants was carried out in order to assess the involvement of gene expression in resistance. On average, expression levels were similar in resistant and susceptible plants.

The presence of a transposon-like element found in exon 11 of the *CsKIP2* gene in genomic DNA was also found not to be correlated to expression levels of *CsKIP2.*

The presence of the transposon-like element in genomic DNA was found to be related to resistance of plants to powdery mildew. The resistant plants with the transposon-like element in the genomic DNA showed a deletion of 72 bp in exon 11 in the cDNA, compared to susceptible plants.

Apparently, the mechanism responsible for the correct splicing of RNA (i.e. separating exons from introns), splices the transposon-like element from the RNA, along with a part (i.e. 72 bp) of the coding sequence. After translation of mRNA to protein, the 72 bp deletion mRNA results in a protein with a 24 amino acid residue deletion. The 24 amino acid residue deletion protein product (i.e. *CsKIP2* from resistant plants) is believed to have lost its function as host-factor.

### Example 2: Cucumber germplasm screen for contribution of CsKIP9 allelic variants to resistance/susceptibility

The cDNA sequence of *CsKIP9* of 5 cucumber plants was determined. **Table 2** below summarizes the plant tested and their powdery mildew resistance.

**Table 2: Cucumber plants used for cDNA sequencing of CsKIP9**

| **Cucumber plant** | **Powdery mildew resistant** |
|---|---|
| OK561 | - |
| OK537 | + |
| OK619 | + |
| OK123 | + |
| OK563 | - |

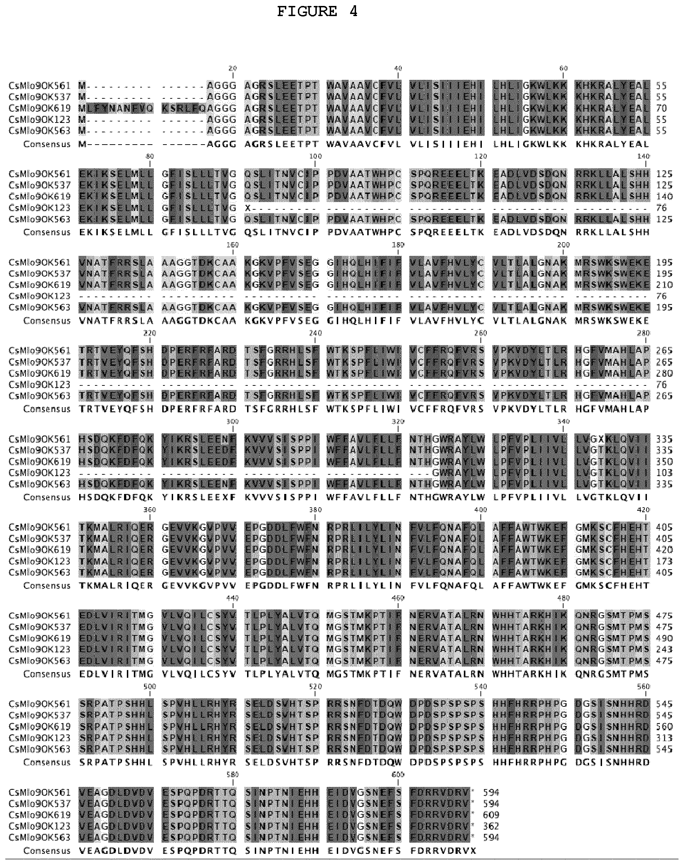

The amino acid sequences encoded by the cDNAs were aligned as shown in **Figure 4****.** An amino acid sunstitution of asparagine (N) by aspartic acid (D) (LEEN to LEED) at position 284 of *CsKIP9* (SEQ ID No. 2) was found to correlate with the powdery mildew resistance observed.

### Example 3: Powdery mildew resistant cucumber plant with non-function CsKIP9.

The cDNA sequence *CsKIP9* of a powdery mildew resistant cucumber plant was determined and an amino acid substation in exon 3 was defined. Specifically, the coding sequence of the first amino acids of exon 3 (positions 61 to 63 of SEQ ID No. 2) of functional *CsKIP9* are Glutamic acid (E) - Leucine (L) - Methionine (M) [ELM]. However, in the powdery mildew resistant cucumber plant identified, this sequence was mutated to Alanine (A) - Threonine (T) - Isoleucine (I) [ATI] indicating that this substitution in *CsKIP9* correlates with the powdery mildew resistance observed.

The powdery mildew resistance providing genes identified herein are summarized in table 3 below. The cDNA and amino acid sequences provided are the powdery mildew resistant genes in their functional form, i.e. providing powdery mildew resistance when impaired at the protein level, such as by mutation, or impaired at the expression level.

**Table 3: cDNA and amino acid sequences of the present genes**

| **gene identity** | **Plant** | **Sequence type** | **SEQ ID No.** |
|---|---|---|---|
| *CsKIP9* | *Cucumis sativus* | cDNA | 1 |
| | | Aa | 2 |
| *CsKIP2* | *Cucumis sativus* | cDNA | 3 |
| | | aa | 4 |
| *CsKIP1* | *Cucumis sativus* | cDNA | 5 |
| | | aa | 6 |
| *CsKIP3* | *Cucumis sativus* | cDNA | 7 |
| | | aa | 8 |
| *CsKIP4* | *Cucumis sativus* | cDNA | 9 |
| | | aa | 10 |
| *CsKIP5* | *Cucumis sativus* | cDNA | 11 |
| | | aa | 12 |
| *CsKIP6* | *Cucumis sativus* | cDNA | 13 |
| | | aa | 14 |
| *CsKIP7* | *Cucumis sativus* | cDNA | 15 |
| | | aa | 16 |
| *CsKIP8* | *Cucumis sativus* | cDNA | 17 |
| | | aa | 18 |
| *CsKIP10* | *Cucumis sativus* | cDNA | 19 |
| | | aa | 20 |
| *CsKIP11* | *Cucumis sativus* | cDNA | 21 |
| | | aa | 22 |

### SEQUENCE LISTING

<110> Enza Zaden Beheer B.V. DIERGAARDE, Paul Johan VAN ENCKEVORT, Leonora Johanna Gertruda POSTHUMA, Karin Ingeborg PRINS, Marinus Willem Keygene N.V.
   <120> POWDERY MILDEW RESISTANCE PROVIDING GENES IN CUCUMIS SATIVUS
   <130> 4/2MF77/31P
   <160> 23
   <170> BiSSAP 1.0
<210> 1
   <211> 1782
   <212> DNA
   <213> Cucumis sativus
<220>
   <221> source
   <222> 1..1782
   <223> /mol_type="DNA" /note="cDNA CsKIP9" /organism="Cucumis sativus"
<400> 1
   atggccggag gtggcgccgg aaggtccttg gaagagacgc cgacatgggc cgtcgccgcc      60
   gtgtgctttg ttttggttct gatttctatt atcatcgaac acattctcca tctcatcgga     120
   aagtggctaa agaagaaaca caaacgagct ctctacgaag ctctggagaa gattaaatca     180
   gaactgatgc tgttgggatt catatcgctg ctgctgacgg tgggacaaag cctaatcaca     240
   aatgtttgta taccacctga cgtggcagcc acgtggcatc catgtagtcc tcaaagagaa     300
   gaagaattaa ctaaagaagc tgacctcgtc gattccgacc aaaatcgtcg aaaacttctc     360
   gccctctccc atcacgtcaa cgccaccttc cgccgttccc tcgccgctgc cggtggtacc     420
   gacaaatgtg ctgccaaggg taaagttcca tttgtatcgg aagggggtat tcatcagcta     480
   catatattca tcttcgtact ggcagttttc catgttttgt attgtgtttt aactttagct     540
   ttgggcaatg ccaagatgag aagttggaag tcatgggaaa aagagacaag aactgtggag     600
   tatcaattct cacacgatcc ggaacggttt cgatttgcaa gagacacgtc atttgggaga     660
   agacacttaa gcttttggac aaaatcccct ttcctcatat ggattgtttg tttcttcaga     720
   caattcgtta ggtcggttcc aaaggttgat tacttgacct taagacatgg tttcgtcatg     780
   gcacatctgg caccgcacag cgatcagaaa tttgactttc aaaaatacat aaaacgatct     840
   cttgaagaaa atttcaaggt ggtggtcagt atcagccctc cgatatggtt ctttgctgtc     900
   ctcttcctac ttttcaacac ccacgggtgg agggcttatc tatggctacc ctttgttccg     960
   ttaattatag tgttattggt ggggacaaag ttgcaagtga taataacgaa aatggcgctg    1020
   aggatacaag aaagaggaga agtggtgaaa ggagtgccgg tggtagagcc aggggatgac    1080
   cttttttggt tcaatcgccc tcgtcttatt ctttacctta tcaattttgt cctcttccag    1140
   aatgcctttc agcttgcctt ttttgcttgg acttggaaag aatttgggat gaaatcttgt    1200
   ttccatgagc acacagagga tttggtcatc agaataacaa tgggggttct cgttcaaatc    1260
   ctttgcagtt atgtcacact gccactttac gctctagtca cacagatggg ttcgacgatg    1320
   aagcccacga ttttcaacga aagagtagcg acggcgttga gaaactggca ccacactgct    1380
   cgtaaacaca taaaacaaaa tcgtggctca atgacgccga tgtcgagccg ccctgcaacc    1440
   ccctcccacc acttgtcacc cgtccacctc cttcgccact atcgaagcga attagatagc    1500
   gttcatacgt ctcctagaag atccaatttc gacaccgatc agtgggaccc tgattcccct    1560
   tccccttccc cttcccacca ctttcaccgc cgtccccatc ccggcgacgg ctccatttcc    1620
   aaccatcacc gtgatgtgga ggccggggat cttgatgtcg atgttgaatc gcctcaaccc    1680
   gaccgaacga cccagtcaat aaacccaaca aatattgagc accatgaaat tgacgtgggg    1740
   tctaacgaat tctcattcga tagaagagtt gatagagtat aa                      1782
<210> 2
   <211> 593
   <212> PRT
   <213> Cucumis sativus
<220>
   <221> SOURCE
   <222> 1..593
   <223> /mol_type="protein" /note="protein CSKIP9" /organism="Cucumis sativus"
<400> 2
   Met Ala Gly Gly Gly Ala Gly Arg Ser Leu Glu Glu Thr Pro Thr Trp
   1               5                   10                  15
   Ala Val Ala Ala Val Cys Phe Val Leu Val Leu Ile Ser Ile Ile Ile
               20                  25                  30
   Glu His Ile Leu His Leu Ile Gly Lys Trp Leu Lys Lys Lys His Lys
           35                  40                  45
   Arg Ala Leu Tyr Glu Ala Leu Glu Lys Ile Lys Ser Glu Leu Met Leu
       50                  55                  60
   Leu Gly Phe Ile Ser Leu Leu Leu Thr Val Gly Gln Ser Leu Ile Thr
   65                  70                  75                  80
   Asn Val Cys Ile Pro Pro Asp Val Ala Ala Thr Trp His Pro Cys Ser
                   85                  90                  95
   Pro Gln Arg Glu Glu Glu Leu Thr Lys Glu Ala Asp Leu Val Asp Ser
               100                 105                 110
   Asp Gln Asn Arg Arg Lys Leu Leu Ala Leu Ser His His Val Asn Ala
           115                 120                 125
   Thr Phe Arg Arg Ser Leu Ala Ala Ala Gly Gly Thr Asp Lys Cys Ala
       130                 135                 140
   Ala Lys Gly Lys Val Pro Phe Val Ser Glu Gly Gly Ile His Gln Leu
   145                 150                 155                 160
   His Ile Phe Ile Phe Val Leu Ala Val Phe His Val Leu Tyr Cys Val
                   165                 170                 175
   Leu Thr Leu Ala Leu Gly Asn Ala Lys Met Arg Ser Trp Lys Ser Trp
               180                 185                 190
   Glu Lys Glu Thr Arg Thr Val Glu Tyr Gln Phe Ser His Asp Pro Glu
           195                 200                 205
   Arg Phe Arg Phe Ala Arg Asp Thr Ser Phe Gly Arg Arg His Leu Ser
       210                 215                 220
   Phe Trp Thr Lys Ser Pro Phe Leu Ile Trp Ile Val Cys Phe Phe Arg
   225                 230                 235                 240
   Gln Phe Val Arg Ser Val Pro Lys Val Asp Tyr Leu Thr Leu Arg His
                   245                 250                 255
   Gly Phe Val Met Ala His Leu Ala Pro His Ser Asp Gln Lys Phe Asp
               260                 265                 270
   Phe Gln Lys Tyr Ile Lys Arg Ser Leu Glu Glu Asn Phe Lys Val Val
           275                 280                 285
   Val Ser Ile Ser Pro Pro Ile Trp Phe Phe Ala Val Leu Phe Leu Leu
       290                 295                 300
   Phe Asn Thr His Gly Trp Arg Ala Tyr Leu Trp Leu Pro Phe Val Pro
   305                 310                 315                 320
   Leu Ile Ile Val Leu Leu Val Gly Thr Lys Leu Gln Val Ile Ile Thr
                   325                 330                 335
   Lys Met Ala Leu Arg Ile Gln Glu Arg Gly Glu Val Val Lys Gly Val
               340                 345                 350
   Pro Val Val Glu Pro Gly Asp Asp Leu Phe Trp Phe Asn Arg Pro Arg
           355                 360                 365
   Leu Ile Leu Tyr Leu Ile Asn Phe Val Leu Phe Gln Asn Ala Phe Gln
       370                 375                 380
   Leu Ala Phe Phe Ala Trp Thr Trp Lys Glu Phe Gly Met Lys Ser Cys
   385                 390                 395                 400
   Phe His Glu His Thr Glu Asp Leu Val Ile Arg Ile Thr Met Gly Val
                   405                 410                 415
   Leu Val Gln Ile Leu Cys Ser Tyr Val Thr Leu Pro Leu Tyr Ala Leu
               420                 425                 430
   Val Thr Gln Met Gly Ser Thr Met Lys Pro Thr Ile Phe Asn Glu Arg
           435                 440                 445
   Val Ala Thr Ala Leu Arg Asn Trp His His Thr Ala Arg Lys His Ile
       450                 455                 460
   Lys Gln Asn Arg Gly Ser Met Thr Pro Met Ser Ser Arg Pro Ala Thr
   465                 470                 475                 480
   Pro Ser His His Leu Ser Pro Val His Leu Leu Arg His Tyr Arg Ser
                   485                 490                 495
   Glu Leu Asp Ser Val His Thr Ser Pro Arg Arg Ser Asn Phe Asp Thr
               500                 505                 510
   Asp Gln Trp Asp Pro Asp Ser Pro Ser Pro Ser Pro Ser His His Phe
           515                 520                 525
   His Arg Arg Pro His Pro Gly Asp Gly Ser Ile Ser Asn His His Arg
       530                 535                 540
   Asp Val Glu Ala Gly Asp Leu Asp Val Asp Val Glu Ser Pro Gln Pro
   545                 550                 555                 560
   Asp Arg Thr Thr Gln Ser Ile Asn Pro Thr Asn Ile Glu His His Glu
                   565                 570                 575
   Ile Asp Val Gly Ser Asn Glu Phe Ser Phe Asp Arg Arg Val Asp Arg
               580                 585                 590
   Val
<210> 3
   <211> 1725
   <212> DNA
   <213> Cucumis sativus
<220>
   <221> source
   <222> 1..1725
   <223> /mol_type="DNA" /note="cDNA CsKIP2" /organism="Cucumis sativus"
<400> 3
   atggctgaat gtggaacaga gcagcgtact ttggaagata cctcaacttg ggctgttgcg      60
   gttgtttgtt ttttcttggt tgttatttca atcttcattg aacatgtcat tcacctcact     120
   ggaaagtggc tggagaaaag gcacaagcca gctcttgttg aagctctaga aaaggttaaa     180
   gcagagctta tgctattggg attcatatcc ctacttctaa cgataggcca agatgctgtc     240
   actcaaattt gtgtttcgaa agagcttgca gcaacttggc ttccctgtgc agcaagagct     300
   aaaacaggag taaaagttgc gaagaacagt cgtcttagac ttcttgaatt tttagatcct     360
   gactatggtt cgaggcgtat tttagcctcg aaaggagatg atgcatgcgc taagaggggc     420
   caactcgctt tcgtgtcggc atatggaatc catcagctcc atattttcat cttcgtattg     480
   gctgtcttcc atgtcctgta ctgcatcata actttggctt ttggcagaac aaagatgagc     540
   aaatggaagg cctgggagga tgaaaccaag acaattgaat accagtacta taatgatcca     600
   gcaagattta gatttgctag agatactacg tttggacgcc gacacttgag cttctggagt     660
   cgtacaccaa tttccctctg gattgtttgt ttcttcagac agttctttgg atcagttacc     720
   aaggttgatt acatgacact gagacatgga ttcattgttg cacatcttgc acccggaagt     780
   gaagtaaaat ttgatttcca caaatacatt agcagatctc tggaagacga ctttaaagtt     840
   gttgtgggga ttagtcccgc aatgtggcta tttgctgttc tcttcatcct aaccaataca     900
   aatgggtggt attcatatct atggctgcct ttcatctcct taattataat tctattggtg     960
   ggaacaaagc tccatgttat tataactcat atgggattga caattcaaga aaggggtcat    1020
   gttgtgaagg gtgttcccgt cgttcagcct cgggatgacc tgttttggtt tggacgtcca    1080
   caacttattc tcttcctgat ccactttgtt ctctttatga atgcatttca gcttgccttc    1140
   tttgcttgga ccacatatgc atttaagtgg atgggttgtt tccatcagcg agttgaagat    1200
   attgtcatca gactctcaat gggggttatc atacaagttc tctgcagtta tgtcacactc    1260
   ccactctatg ctttggttac tcagatgggc tctaacatga gaccaaccat tttcaacgac    1320
   cgagtggcga cggcattgaa gaactggcac cactcagcca agaagaacat gaagcagcac    1380
   cgcaacccag acagtacctc accattctca agcaggccag ctactccaac tcacggcatg    1440
   tctcctattc accttctgca caaacatcag catggcagca catctcccag gctatccgat    1500
   gccgaacccg atcgttggga agagttgcct ccttcttcac accatagtag agccccccat     1560
   catgataatc atcaagatca acaagaacaa tctgagacaa taattagaga acaggagatg    1620
   acagttcaag gaccaagttc aagtgaaacc ggttccataa cacgtcctgc tcgccctcat    1680
   caggaaatca ctaggactcc atcagacttc tcatttgcca aatga                    1725
<210> 4
   <211> 574
   <212> PRT
   <213> Cucumis sativus
<220>
   <221> SOURCE
   <222> 1..574
   <223> /mol_type="protein" /note="protein CsKIP2" /organism="Cucumis sativus"
<400> 4
   Met Ala Glu Cys Gly Thr Glu Gln Arg Thr Leu Glu Asp Thr Ser Thr
   1               5                   10                  15
   Trp Ala Val Ala Val Val Cys Phe Phe Leu Val Val Ile Ser Ile Phe
               20                  25                  30
   Ile Glu His Val Ile His Leu Thr Gly Lys Trp Leu Glu Lys Arg His
           35                  40                  45
   Lys Pro Ala Leu Val Glu Ala Leu Glu Lys Val Lys Ala Glu Leu Met
       50                  55                  60
   Leu Leu Gly Phe Ile Ser Leu Leu Leu Thr Ile Gly Gln Asp Ala Val
   65                  70                  75                  80
   Thr Gln Ile Cys Val Ser Lys Glu Leu Ala Ala Thr Trp Leu Pro Cys
                   85                  90                  95
   Ala Ala Arg Ala Lys Thr Gly Val Lys Val Ala Lys Asn Ser Arg Leu
               100                 105                 110
   Arg Leu Leu Glu Phe Leu Asp Pro Asp Tyr Gly Ser Arg Arg Ile Leu
           115                 120                 125
   Ala Ser Lys Gly Asp Asp Ala Cys Ala Lys Arg Gly Gln Leu Ala Phe
       130                 135                 140
   Val Ser Ala Tyr Gly Ile His Gln Leu His Ile Phe Ile Phe Val Leu
   145                 150                 155                 160
   Ala Val Phe His Val Leu Tyr Cys Ile Ile Thr Leu Ala Phe Gly Arg
                   165                 170                 175
   Thr Lys Met Ser Lys Trp Lys Ala Trp Glu Asp Glu Thr Lys Thr Ile
               180                 185                 190
   Glu Tyr Gln Tyr Tyr Asn Asp Pro Ala Arg Phe Arg Phe Ala Arg Asp
           195                 200                 205
   Thr Thr Phe Gly Arg Arg His Leu Ser Phe Trp Ser Arg Thr Pro Ile
       210                 215                 220
   Ser Leu Trp Ile Val Cys Phe Phe Arg Gln Phe Phe Gly Ser Val Thr
   225                 230                 235                 240
   Lys Val Asp Tyr Met Thr Leu Arg His Gly Phe Ile Val Ala His Leu
                   245                 250                 255
   Ala Pro Gly Ser Glu Val Lys Phe Asp Phe His Lys Tyr Ile Ser Arg
               260                 265                 270
   Ser Leu Glu Asp Asp Phe Lys Val Val Val Gly Ile Ser Pro Ala Met
           275                 280                 285
   Trp Leu Phe Ala Val Leu Phe Ile Leu Thr Asn Thr Asn Gly Trp Tyr
       290                 295                 300
   Ser Tyr Leu Trp Leu Pro Phe Ile Ser Leu Ile Ile Ile Leu Leu Val
   305                 310                 315                     320
   Gly Thr Lys Leu His Val Ile Ile Thr His Met Gly Leu Thr Ile Gln
                   325                 330                 335
   Glu Arg Gly His Val Val Lys Gly Val Pro Val Val Gln Pro Arg Asp
               340                 345                 350
   Asp Leu Phe Trp Phe Gly Arg Pro Gln Leu Ile Leu Phe Leu Ile His
           355                 360                 365
   Phe Val Leu Phe Met Asn Ala Phe Gln Leu Ala Phe Phe Ala Trp Thr
       370                 375                 380
   Thr Tyr Ala Phe Lys Trp Met Gly Cys Phe His Gln Arg Val Glu Asp
   385                 390                 395                     400
   Ile Val Ile Arg Leu Ser Met Gly Val Ile Ile Gln Val Leu Cys Ser
                   405                 410                 415
   Tyr Val Thr Leu Pro Leu Tyr Ala Leu Val Thr Gln Met Gly Ser Asn
               420                 425                 430
   Met Arg Pro Thr Ile Phe Asn Asp Arg Val Ala Thr Ala Leu Lys Asn
           435                 440                 445
   Trp His His Ser Ala Lys Lys Asn Met Lys Gln His Arg Asn Pro Asp
       450                 455                 460
   Ser Thr Ser Pro Phe Ser Ser Arg Pro Ala Thr Pro Thr His Gly Met
   465                 470                 475                     480
   Ser Pro Ile His Leu Leu His Lys His Gln His Gly Ser Thr Ser Pro
                   485                 490                 495
   Arg Leu Ser Asp Ala Glu Pro Asp Arg Trp Glu Glu Leu Pro Pro Ser
               500                 505                 510
   Ser His His Ser Arg Ala Pro His His Asp Asn His Gln Asp Gln Gln
           515                 520                 525
   Glu Gln Ser Glu Thr Ile Ile Arg Glu Gln Glu Met Thr Val Gln Gly
       530                 535                 540
   Pro Ser Ser Ser Glu Thr Gly Ser Ile Thr Arg Pro Ala Arg Pro His
   545                 550                 555                     560
   Gln Glu Ile Thr Arg Thr Pro Ser Asp Phe Ser Phe Ala Lys
                   565                 570
<210> 5
   <211> 1749
   <212> DNA
   <213> Cucumis sativus
<220>
   <221> source
   <222> 1..1749
   <223> /mol_type="DNA" /note="cDNA CsKIP1" /organism="Cucumis sativus"
<400> 5
   atggcggggg cagccggtgg caagtcgctg gagcaaacac cgacatgggc cgttgccgtt      60
   gtttgctttg ttttgctcgt catctctatt ttcatcgaat atagtctcca tcttatcgga     120
   cattggctaa agaagagaca caaacgggcg ttgtttgaag cattagagaa gatcaaatca     180
   gagcttatgt tattggggtt tatatcattg ctactaacgg tggggcaagg accaataacg     240
   gagatatgta ttccacaaca tgtagctgca acgtggcatc catgtacaaa ggaaagagaa      300
   gatgagatga acaaagaggt ggagaaatct gtggaacatt tgggtcttga tcgccggaga      360
   ctccttcatc tcctcggaaa tggtgaaagt ttccggcgga gtttggccgc tgcgggagga      420
   gaggataaat gtgccgccaa gggtaaagct tcctttattt cagcagatgg aattcatcaa      480
   cttcatatct tcatttttgt gttggcagtt tttcatgttt tgtattgtgt tctaacttat      540
   gcgttggcta gagctaagat gaggagttgg aaaacatggg aaaaagagac caaaactgct      600
   gaataccaat tctcacatga tccagagagg tttaggtttg caagagacac ctcatttggg      660
   agaagacatt tgagcttttg gaccaaaaat cctgccttga tgtggatcgt tcgtttcttc      720
   agacaatttg taagatctgt tccaaaagtt gattacttga cattaagaca tgggtttata      780
   atggcacatt tagcacctca aagtcttaca caatttgatt ttcaaaaata cattaataga      840
   tcccttgaag aagacttcaa agttgttgtg ggaatcagcc cgccaatttg gttctttgct      900
   gttctatctc tcctctcaaa cactcacggt tggagggcgt atctatggct gccattcatc      960
   ccactaatca ttttgctgtt gattggaaca aaattgcaag tgatcataac gaaaatggca     1020
   ctaagaatac aagaaagagg tgaagtagtg aagggcgtgc cggtggtgga gcctggcggt     1080
   gacctctttt ggtttaatcg gcctcgcctt attctttatc tcatcaactt tgttctcttt     1140
   caaaatgcct tccaagttgc cttctttgct tggacttggt atgagtttgg gttgaattct     1200
   tgcttccatg agcatataga agatgtggtg atcagaattt ctatgggggt gcttgtacaa     1260
   atcctttgca gttatgttac tcttcctctt tatgcactag tcactcagat gggttcaaca     1320
   atgaagccaa ctatattcaa tgagagagtg gcagaggccc ttcgcaattg gtaccactcg     1380
   gctcgaaagc acatcaaaca caaccgcggt tcggtcactc caatgtcgag ccgacccgcc     1440
   accccgactc acagcatgtc gcctgtccac cttctccgac actacaagag tgaagtcgat     1500
   agcttccaca cctcaccgag aaggtcaccg ttcgacaccg atcgttggga caacgattcg     1560
   ccctctccat ctcgccatgt tgatggttcg tcttcgtcac aaccccacgt tgagatggga     1620
   ggttatgaaa aagatcccgt tgaatcaagt tcgtctcgag ttgatccggt tcaaccatct     1680
   cgaaaccgca atcaacatga gattcatatt ggaggcccca aagacttttc atttgataga     1740
   gttgaatga                                                             1749
<210> 6
   <211> 582
   <212> PRT
   <213> Cucumis sativus
<220>
   <221> SOURCE
   <222> 1..582
   <223> /mol_type="protein" /note="protein CsKIP1" /organism="Cucumis sativus"
<400> 6
   Met Ala Gly Ala Ala Gly Gly Lys Ser Leu Glu Gln Thr Pro Thr Trp
   1               5                   10                  15
   Ala Val Ala Val Val Cys Phe Val Leu Leu Val Ile Ser Ile Phe Ile
               20                  25                  30
   Glu Tyr Ser Leu His Leu Ile Gly His Trp Leu Lys Lys Arg His Lys
           35                  40                  45
   Arg Ala Leu Phe Glu Ala Leu Glu Lys Ile Lys Ser Glu Leu Met Leu
       50                  55                  60
   Leu Gly Phe Ile Ser Leu Leu Leu Thr Val Gly Gln Gly Pro Ile Thr
   65                  70                  75                  80
   Glu Ile Cys Ile Pro Gln His Val Ala Ala Thr Trp His Pro Cys Thr
                   85                  90                  95
   Lys Glu Arg Glu Asp Glu Met Asn Lys Glu Val Glu Lys Ser Val Glu
               100                 105                 110
   His Leu Gly Leu Asp Arg Arg Arg Leu Leu His Leu Leu Gly Asn Gly
           115                 120                 125
   Glu Ser Phe Arg Arg Ser Leu Ala Ala Ala Gly Gly Glu Asp Lys Cys
       130                 135                 140
   Ala Ala Lys Gly Lys Ala Ser Phe Ile Ser Ala Asp Gly Ile His Gln
   145                 150                 155                 160
   Leu His Ile Phe Ile Phe Val Leu Ala Val Phe His Val Leu Tyr Cys
                   165                 170                 175
   Val Leu Thr Tyr Ala Leu Ala Arg Ala Lys Met Arg Ser Trp Lys Thr
               180                 185                 190
   Trp Glu Lys Glu Thr Lys Thr Ala Glu Tyr Gln Phe Ser His Asp Pro
           195                 200                 205
   Glu Arg Phe Arg Phe Ala Arg Asp Thr Ser Phe Gly Arg Arg His Leu
       210                 215                 220
   Ser Phe Trp Thr Lys Asn Pro Ala Leu Met Trp Ile Val Arg Phe Phe
   225                 230                 235                 240
   Arg Gln Phe Val Arg Ser Val Pro Lys Val Asp Tyr Leu Thr Leu Arg
                   245                 250                 255
   His Gly Phe Ile Met Ala His Leu Ala Pro Gln Ser Leu Thr Gln Phe
               260                 265                 270
   Asp Phe Gln Lys Tyr Ile Asn Arg Ser Leu Glu Glu Asp Phe Lys Val
           275                 280                 285
   Val Val Gly Ile Ser Pro Pro Ile Trp Phe Phe Ala Val Leu Ser Leu
       290                 295                 300
   Leu Ser Asn Thr His Gly Trp Arg Ala Tyr Leu Trp Leu Pro Phe Ile
   305                 310                 315                 320
   Pro Leu Ile Ile Leu Leu Leu Ile Gly Thr Lys Leu Gln Val Ile Ile
                   325                 330                 335
   Thr Lys Met Ala Leu Arg Ile Gln Glu Arg Gly Glu Val Val Lys Gly
               340                 345                 350
   Val Pro Val Val Glu Pro Gly Gly Asp Leu Phe Trp Phe Asn Arg Pro
           355                 360                 365
   Arg Leu Ile Leu Tyr Leu Ile Asn Phe Val Leu Phe Gln Asn Ala Phe
       370                 375                 380
   Gln Val Ala Phe Phe Ala Trp Thr Trp Tyr Glu Phe Gly Leu Asn Ser
   385                 390                 395                 400
   Cys Phe His Glu His Ile Glu Asp Val Val Ile Arg Ile Ser Met Gly
                   405                 410                 415
   Val Leu Val Gln Ile Leu Cys Ser Tyr Val Thr Leu Pro Leu Tyr Ala
               420                 425                 430
   Leu Val Thr Gln Met Gly Ser Thr Met Lys Pro Thr Ile Phe Asn Glu
           435                 440                 445
   Arg Val Ala Glu Ala Leu Arg Asn Trp Tyr His Ser Ala Arg Lys His
       450                 455                 460
   Ile Lys His Asn Arg Gly Ser Val Thr Pro Met Ser Ser Arg Pro Ala
   465                 470                 475                 480
   Thr Pro Thr His Ser Met Ser Pro Val His Leu Leu Arg His Tyr Lys
                   485                 490                 495
   Ser Glu Val Asp Ser Phe His Thr Ser Pro Arg Arg Ser Pro Phe Asp
               500                 505                 510
   Thr Asp Arg Trp Asp Asn Asp Ser Pro Ser Pro Ser Arg His Val Asp
           515                 520                 525
   Gly Ser Ser Ser Ser Gln Pro His Val Glu Met Gly Gly Tyr Glu Lys
       530                 535                 540
   Asp Pro Val Glu Ser Ser Ser Ser Arg Val Asp Pro Val Gln Pro Ser
   545                 550                 555                 560
   Arg Asn Arg Asn Gln His Glu Ile His Ile Gly Gly Pro Lys Asp Phe
                   565                 570                 575
   Ser Phe Asp Arg Val Glu
               580
<210> 7
   <211> 1551
   <212> DNA
   <213> Cucumis sativus
<220>
   <221> source
   <222> 1..1551
   <223> /mol_type="DNA" /note="cDNA CsKIP3" /organism="Cucumis sativus"
<400> 7
   atgggtggcg gaggtgaagg aacgacgctg gaattcactc cgacgtgggt tgtagccgcc      60
   gtatgtactg tcatcgttgc catttctctc gccttagagc gccttcttca ctttctcggc     120
   agatacctca aaagcaagaa tcaaaagccg ctcaatgaag ctcttcagaa agttaaagaa     180
   gaattgatgc ttttggggtt catttcactt ctgctcactg tatttcaagg caccatctct     240
   aaattgtgtg ttcctgagag tttgactgaa catttacttc cgtgtgatct gaaggataaa     300
   ccgaaagctg aacatggttc gccctctggt gaatctggtt cgtcaacgac gaagcatttt     360
   caaacgttat ttgtttcgag tatttctggt acggccaggc ggcttctttc tgagggatct     420
   gcttcacaag ctggttactg tgccaaaaag aataaggtgc cattgctatc tcttgaagca     480
   ttgcatcatc tacatatttt tatcttcatc ctagctatcg tccacgtgac attttgcgtt     540
   ctcactgtag tttttggagg attgaagatt cgccagcgga agcattggga ggattctatt     600
   gcaaaagaga attatgatac tgaacaagtt ctaaaaccaa aggtcactca tgtccatcaa     660
   catgttttta tcaaagacca ctttttgggc tttggtaaag attcagctct tcttggttgg     720
   ttgcattcat ttctcaagca attttatgct tctgtaacaa aatcagatta tgcaacgtta     780
   cgacttggtt tcattacgac gcactgcagg ggaaatccaa agtttaattt tcacaagtac      840
   atgatacgtg cccttgaaga tgacttcaag catgttgttg gtatcagttg gtatctttgg      900
   atattcgtgg ttgtcttctt gttccttaat gtcagtggtt ggcatacata tttctggata      960
   gcattcattc ctttcgttct tctgcttgct gtgggaacga agctggaaca tgtgataacc     1020
   cagctggctc atgaggttgc agagaagcac atagcaatcg aaggtgatct agtagtccaa     1080
   ccgtctgatg atcacttttg gttccaacgt ccccgtattg ttctcttctt gatccacttt     1140
   atacttttcc aaaacgcttt tgagattgga tttttcttct ggatatgggt tcaatatgga     1200
   tttgactcgt gcatcatggg acaagtccgc tatatcattc caaggctcat cattggggtg     1260
   tttgttcagg ttctttgcag ttacagcacc cttctgctct gcgccattgt cactcagatg     1320
   ggaagttctt tcaagaaagc aatctttgat gaacatgtac aagtagggct agttggctgg     1380
   gctcagaagg tgaagaaaag aaagggactt agagcagctg ctgatggctc cagtcaagga     1440
   gtcaaggaag gtggttcaac tgtggggatt cagttgggaa atgttatgcg caaggcttct     1500
   gcacctcaag aaattaagcc tgatgactcc aaatcaaatg atattcctta g              1551
<210> 8
   <211> 516
   <212> PRT
   <213> Cucumis sativus
<220>
   <221> SOURCE
   <222> 1..516
   <223> /mol_type="protein" /note="protein CsKIP3" /organism="Cucumis sativus"
<400> 8
   Met Gly Gly Gly Gly Glu Gly Thr Thr Leu Glu Phe Thr Pro Thr Trp
   1               5                   10                  15
   Val Val Ala Ala Val Cys Thr Val Ile Val Ala Ile Ser Leu Ala Leu
               20                  25                  30
   Glu Arg Leu Leu His Phe Leu Gly Arg Tyr Leu Lys Ser Lys Asn Gln
           35                  40                  45
   Lys Pro Leu Asn Glu Ala Leu Gln Lys Val Lys Glu Glu Leu Met Leu
       50                  55                  60
   Leu Gly Phe Ile Ser Leu Leu Leu Thr Val Phe Gln Gly Thr Ile Ser
   65                  70                  75                  80
   Lys Leu Cys Val Pro Glu Ser Leu Thr Glu His Leu Leu Pro Cys Asp
                   85                  90                  95
   Leu Lys Asp Lys Pro Lys Ala Glu His Gly Ser Pro Ser Gly Glu Ser
               100                 105                 110
   Gly Ser Ser Thr Thr Lys His Phe Gln Thr Leu Phe Val Ser Ser Ile
           115                 120                 125
   Ser Gly Thr Ala Arg Arg Leu Leu Ser Glu Gly Ser Ala Ser Gln Ala
       130                 135                 140
   Gly Tyr Cys Ala Lys Lys Asn Lys Val Pro Leu Leu Ser Leu Glu Ala
   145                 150                 155                 160
   Leu His His Leu His Ile Phe Ile Phe Ile Leu Ala Ile Val His Val
                   165                 170                 175
   Thr Phe Cys Val Leu Thr Val Val Phe Gly Gly Leu Lys Ile Arg Gln
               180                 185                 190
   Arg Lys His Trp Glu Asp Ser Ile Ala Lys Glu Asn Tyr Asp Thr Glu
           195                 200                 205
   Gln Val Leu Lys Pro Lys Val Thr His Val His Gln His Val Phe Ile
       210                 215                 220
   Lys Asp His Phe Leu Gly Phe Gly Lys Asp Ser Ala Leu Leu Gly Trp
   225                 230                 235                 240
   Leu His Ser Phe Leu Lys Gln Phe Tyr Ala Ser Val Thr Lys Ser Asp
                   245                 250                 255
   Tyr Ala Thr Leu Arg Leu Gly Phe Ile Thr Thr His Cys Arg Gly Asn
               260                 265                 270
   Pro Lys Phe Asn Phe His Lys Tyr Met Ile Arg Ala Leu Glu Asp Asp
           275                 280                 285
   Phe Lys His Val Val Gly Ile Ser Trp Tyr Leu Trp Ile Phe Val Val
       290                 295                 300
   Val Phe Leu Phe Leu Asn Val Ser Gly Trp His Thr Tyr Phe Trp Ile
   305                 310                 315                 320
   Ala Phe Ile Pro Phe Val Leu Leu Leu Ala Val Gly Thr Lys Leu Glu
                   325                 330                 335
   His Val Ile Thr Gln Leu Ala His Glu Val Ala Glu Lys His Ile Ala
               340                 345                 350
   Ile Glu Gly Asp Leu Val Val Gln Pro Ser Asp Asp His Phe Trp Phe
           355                 360                 365
   Gln Arg Pro Arg Ile Val Leu Phe Leu Ile His Phe Ile Leu Phe Gln
       370                 375                 380
   Asn Ala Phe Glu Ile Gly Phe Phe Phe Trp Ile Trp Val Gln Tyr Gly
   385                 390                 395                 400
   Phe Asp Ser Cys Ile Met Gly Gln Val Arg Tyr Ile Ile Pro Arg Leu
                   405                 410                 415
   Ile Ile Gly Val Phe Val Gln Val Leu Cys Ser Tyr Ser Thr Leu Leu
               420                 425                 430
   Leu Cys Ala Ile Val Thr Gln Met Gly Ser Ser Phe Lys Lys Ala Ile
           435                 440                 445
   Phe Asp Glu His Val Gln Val Gly Leu Val Gly Trp Ala Gln Lys Val
       450                 455                 460
   Lys Lys Arg Lys Gly Leu Arg Ala Ala Ala Asp Gly Ser Ser Gln Gly
   465                 470                 475                 480
   Val Lys Glu Gly Gly Ser Thr Val Gly Ile Gln Leu Gly Asn Val Met
                   485                 490                 495
   Arg Lys Ala Ser Ala Pro Gln Glu Ile Lys Pro Asp Asp Ser Lys Ser
               500                 505                 510
   Asn Asp Ile Pro
           515
<210> 9
   <211> 1641
   <212> DNA
   <213> Cucumis sativus
<220>
   <221> source
   <222> 1..1641
   <223> /mol_type="DNA" /note="cDNA CsKIP4" /organism="Cucumis sativus"
<400> 9
   atgttgctgg ttgtttatta tttgtgcttg agcttgttgt gggggaaatc gtggggggct      60
   ccggccagcg atggcaccac gagggagctc gatcagactc cgacatgggc tgttgctggt    120
   gtttgtgcta ttattatcct tatttctatc gccttggaga aactccttca taaagctgga    180
   acgtggctca cggaaaagca caagagagct ctctttgaag ctctggagaa agttaaagct    240
   gagctgatga ttctgggttt catttcactg ctcctcacct ttggacagaa ctacatcatt    300
   aaaatatgca ttcccacaaa ggttgcaaat actatgttgc catgtgctgc caaagaggac    360
   aaattggaga agggagatga aggcgaacat catcgacgac ttctaatgta tgaacggagg    420
   ttcctggctg ctgctggtgg cgctgttagt tgcaaagaag gtcatgtgcc gcttatatct    480
   atctcgggat tgcatcagtt gcacttgttt atcttcttct tagccgtatt tcatgtggta    540
   tatagtgcca tcacaatgat gcttgggagg ctaaagattc gaggttggaa ggcatgggag    600
   gaggagacct caactcacaa ttatgagttc tcaaatgata atgcacgatt caggcttact    660
   cacgaaacat catttgtgaa agcccacacg agtttttgga caaaacttcc cgtcttcttt    720
   tatattggat gcttcttccg acaatttttc aagtccgttg gtcaccttgc tccgggaagt    780
   aaatttgact ttcaaaaata tatcaaaagg tctctagaag atgacttcaa aataattgtg    840
   ggagttagtc ccgtgctttg gacatcgttt gtggtcttct tgctcataaa tgtttacgga    900
   tggcaagcat tgttttggtc atccttagtt cctgtgatca taatcctagc tgttggaacc    960
   aagcttcaag gagttatgac aaagatggct ctcgaaatta cagaaagaca tgctgttgtc   1020
   caaggaattc ctctcgttca ggcatcagat aaatattttt ggtttggcaa gcctcagctg   1080
   gttctttacc tcatccactt cgctttattt tcgaatgcat tccaaataac atacttcttc   1140
   tggatttggt attcctttgg gttaaaatcc tgcttccata ctgatttcaa gctcgcaatc   1200
   attaaagttg gtctcggggt tggcgttctc tgtctctgca gttatataac tcttccactc   1260
   tatgctcttg tcactcagat gggtacgcgt atgaagaagt caatctttga tgaacaaaca   1320
   tcgaaggctc ttaagaagtg gcacatggct gttaagaagc gacatgggaa gtccccaact   1380
   cgaaaactag ggagtccaag ttcatcacca attcatccat catcaggata cgcattgcat   1440
   cgtttcaaga ccacaggtca ctcgaacaga tcatccatgt atgatgagaa tgacgcatca   1500
   gattatgaag tcgacactcc aaattttaca gttagaatag accatggtga tgaacatcaa   1560
   gctgaaataa ttgaacccca gcatacagaa aaaaggaatg aagacgattt ctcttttgtc   1620
   aaacctggac carsgaaatg a                                              1641
<210> 10
   <211> 546
   <212> PRT
   <213> Cucumis sativus
<220>
   <221> SOURCE
   <222> 1..546
   <223> /mol_type="protein" /note="proetin CsKIP4" /organism="Cucumis sativus"
<400> 10
   Met Leu Leu Val Val Tyr Tyr Leu Cys Leu Ser Leu Leu Trp Gly Lys
   1               5                   10                  15
   Ser Trp Gly Ala Pro Ala Ser Asp Gly Thr Thr Arg Glu Leu Asp Gln
               20                  25                  30
   Thr Pro Thr Trp Ala Val Ala Gly Val Cys Ala Ile Ile Ile Leu Ile
           35                  40                  45
   Ser Ile Ala Leu Glu Lys Leu Leu His Lys Ala Gly Thr Trp Leu Thr
       50                  55                  60
   Glu Lys His Lys Arg Ala Leu Phe Glu Ala Leu Glu Lys Val Lys Ala
   65                  70                  75                  80
   Glu Leu Met Ile Leu Gly Phe Ile Ser Leu Leu Leu Thr Phe Gly Gln
                   85                  90                  95
   Asn Tyr Ile Ile Lys Ile Cys Ile Pro Thr Lys Val Ala Asn Thr Met
               100                 105                 110
   Leu Pro Cys Ala Ala Lys Glu Asp Lys Leu Glu Lys Gly Asp Glu Gly
           115                 120                 125
   Glu His His Arg Arg Leu Leu Met Tyr Glu Arg Arg Phe Leu Ala Ala
       130                 135                 140
   Ala Gly Gly Ala Val Ser Cys Lys Glu Gly His Val Pro Leu Ile Ser
   145                 150                 155                 160
   Ile Ser Gly Leu His Gln Leu His Leu Phe Ile Phe Phe Leu Ala Val
                   165                 170                 175
   Phe His Val Val Tyr Ser Ala Ile Thr Met Met Leu Gly Arg Leu Lys
               180                 185                 190
   Ile Arg Gly Trp Lys Ala Trp Glu Glu Glu Thr Ser Thr His Asn Tyr
           195                 200                 205
   Glu Phe Ser Asn Asp Asn Ala Arg Phe Arg Leu Thr His Glu Thr Ser
       210                 215                 220
   Phe Val Lys Ala His Thr Ser Phe Trp Thr Lys Leu Pro Val Phe Phe
   225                 230                 235                 240
   Tyr Ile Gly Cys Phe Phe Arg Gln Phe Phe Lys Ser Val Gly His Leu
                   245                 250                 255
   Ala Pro Gly Ser Lys Phe Asp Phe Gln Lys Tyr Ile Lys Arg Ser Leu
               260                 265                 270
   Glu Asp Asp Phe Lys Ile Ile Val Gly Val Ser Pro Val Leu Trp Thr
           275                 280                 285
   Ser Phe Val Val Phe Leu Leu Ile Asn Val Tyr Gly Trp Gln Ala Leu
       290                 295                 300
   Phe Trp Ser Ser Leu Val Pro Val Ile Ile Ile Leu Ala Val Gly Thr
   305                 310                 315                 320
   Lys Leu Gln Gly Val Met Thr Lys Met Ala Leu Glu Ile Thr Glu Arg
                   325                 330                 335
   His Ala Val Val Gln Gly Ile Pro Leu Val Gln Ala Ser Asp Lys Tyr
               340                 345                 350
   Phe Trp Phe Gly Lys Pro Gln Leu Val Leu Tyr Leu Ile His Phe Ala
           355                 360                 365
   Leu Phe Ser Asn Ala Phe Gln Ile Thr Tyr Phe Phe Trp Ile Trp Tyr
       370                 375                 380
   Ser Phe Gly Leu Lys Ser Cys Phe His Thr Asp Phe Lys Leu Ala Ile
   385                 390                 395                 400
   Ile Lys Val Gly Leu Gly Val Gly Val Leu Cys Leu Cys Ser Tyr Ile
                   405                 410                 415
   Thr Leu Pro Leu Tyr Ala Leu Val Thr Gln Met Gly Thr Arg Met Lys
               420                 425                 430
   Lys Ser Ile Phe Asp Glu Gln Thr Ser Lys Ala Leu Lys Lys Trp His
           435                 440                 445
   Met Ala Val Lys Lys Arg His Gly Lys Ser Pro Thr Arg Lys Leu Gly
       450                 455                 460
   Ser Pro Ser Ser Ser Pro Ile His Pro Ser Ser Gly Tyr Ala Leu His
   465                 470                 475                 480
   Arg Phe Lys Thr Thr Gly His Ser Asn Arg Ser Ser Met Tyr Asp Glu
                   485                 490                 495
   Asn Asp Ala Ser Asp Tyr Glu Val Asp Thr Pro Asn Phe Thr Val Arg
               500                 505                 510
   Ile Asp His Gly Asp Glu His Gln Ala Glu Ile Ile Glu Pro Gln His
           515                 520                 525
   Thr Glu Lys Arg Asn Glu Asp Asp Phe Ser Phe Val Lys Pro Gly Pro
       530                 535                 540
   Thr Lys
   545
<210> 11
   <211> 1638
   <212> DNA
   <213> Cucumis sativus
<220>
   <221> source
   <222> 1..1638
   <223> /mol_type="DNA" /note="cDNA CsKIP5" /organism="Cucumis sativus"
<400> 11
   atgggtggcg gtgccggtgc cggtggtccg agtagggagt tagatcaaac tccgacatgg      60
   gccgttgccg ctgtttgtgc agtcatcatt cttatttcca tcatattgga aaaggttctt     120
   cacatggttg gagagatatt tcaaaaaagg aaaaagaaag ccttgtatga agcgctcgag     180
   aaagttaaag gaggggagct tatggtttta ggattcattt ctttgctctt aacatttggg     240
   caaaattata ttgctaaagt ttgtataccc tcaaagtatg aaaatactat gttgccttgc     300
   ccttttagag ggagtagtac tactttacct aaaagctccc atcacgccga gcctgatgat     360
   gatgaagaga cttccgatca ccatcgtagg cttctttggt acgagcatcg acgtctaggt     420
   ggtggtggtt ctgtagaagg ttgcaaacca gggtatacac aacttatatc tctaaatggt     480
   ttgcatcaaa tacatatctt catcttcttt ctagccgttc tccatgttgt atttagtgcc     540
   ataacgatga ctctcggaag attgaaaatt cgtgcgtgga aggtatggga aagacagacc     600
   gaacaagaac atgatgccat gaacgatcct acaaggttta gacttactca cgagacatcc     660
   tttgtgagag atcacagcag tttttggacc aaaacccccc tctcctttta ctttgtatgc     720
   ttctggaggc aattctttag gtccgttagt aggccagatt acttgtccct tagacatggt     780
   tttgtcactg tccatttagc ccctgggagt aaatttgact ttcagaagta cattaaaagg     840
   tcattagaag atgactttaa ggtggtcgtg ggaatcagtc ctctgctatg ggcatcaatg     900
   gtgctttttc tgcttctcaa tgttaatggg tggcaagtta tgttttgggt gtctatattt     960
   cctctagtgg tgatcttagc tgttggaaca aagttgcaag gaattataac acaaatggct    1020
   cttgaaatca aagaaagaca tgcagtggtt caagggattc cccttgttca agtctctgat    1080
   agacattttt ggtttagttg gcccattttg gttctttatc tcatccatta tgtccttttc    1140
   cagaatgcat ttgagattac atatttcttt tggatatggt atgaatttgg gttgagatca    1200
   tgctttcatg acaactttga tcttattatc gcgagagttg gtctaggggt tggagtccag    1260
   attttgtgca gttacattac actcccatta tatgctcttg taactcagat gggatcaaca    1320
   atgaagaaat ccatatttga tgaacaaact tcgaaagcat tgaagcaatg gcatagaagt    1380
   gctttgaaga aaaagaacga aggaggaaag cctgaaccaa cgccgatgcg aactttaggc    1440
   ggtgccgttg ttgttggagg aagccctccc gagtcaccga tacaacaacc tttgcatgat    1500
   caattccaac atcaaacaat gactcaatca tcaccaaccg acgtcgaagc ctccgccgtt    1560
   ccttcagtca acataatgac taccgttgat ctccaccaac aacagcaaaa ctattccaat    1620
   cgtgacttgt tgagatga                                                  1638
<210> 12
   <211> 545
   <212> PRT
   <213> Cucumis sativus
<220>
   <221> SOURCE
   <222> 1..545
   <223> /mol_type="protein" /note="protein CsKIP5" /organism="Cucumis sativus"
<400> 12
   Met Gly Gly Gly Ala Gly Ala Gly Gly Pro Ser Arg Glu Leu Asp Gln
   1               5                   10                  15
   Thr Pro Thr Trp Ala Val Ala Ala Val Cys Ala Val Ile Ile Leu Ile
               20                  25                  30
   Ser Ile Ile Leu Glu Lys Val Leu His Met Val Gly Glu Ile Phe Gln
           35                  40                  45
   Lys Arg Lys Lys Lys Ala Leu Tyr Glu Ala Leu Glu Lys Val Lys Gly
       50                  55                  60
   Gly Glu Leu Met Val Leu Gly Phe Ile Ser Leu Leu Leu Thr Phe Gly
   65                  70                  75                  80
   Gln Asn Tyr Ile Ala Lys Val Cys Ile Pro Ser Lys Tyr Glu Asn Thr
                   85                  90                  95
   Met Leu Pro Cys Pro Phe Arg Gly Ser Ser Thr Thr Leu Pro Lys Ser
               100                 105                 110
   Ser His His Ala Glu Pro Asp Asp Asp Glu Glu Thr Ser Asp His His
           115                 120                 125
   Arg Arg Leu Leu Trp Tyr Glu His Arg Arg Leu Gly Gly Gly Gly Ser
       130                 135                 140
   Val Glu Gly Cys Lys Pro Gly Tyr Thr Gln Leu Ile Ser Leu Asn Gly
   145                 150                 155                 160
   Leu His Gln Ile His Ile Phe Ile Phe Phe Leu Ala Val Leu His Val
                   165                 170                 175
   Val Phe Ser Ala Ile Thr Met Thr Leu Gly Arg Leu Lys Ile Arg Ala
               180                 185                 190
   Trp Lys Val Trp Glu Arg Gln Thr Glu Gln Glu His Asp Ala Met Asn
           195                 200                 205
   Asp Pro Thr Arg Phe Arg Leu Thr His Glu Thr Ser Phe Val Arg Asp
       210                 215                 220
   His Ser Ser Phe Trp Thr Lys Thr Pro Leu Ser Phe Tyr Phe Val Cys
   225                 230                 235                 240
   Phe Trp Arg Gln Phe Phe Arg Ser Val Ser Arg Pro Asp Tyr Leu Ser
                   245                 250                 255
   Leu Arg His Gly Phe Val Thr Val His Leu Ala Pro Gly Ser Lys Phe
               260                 265                 270
   Asp Phe Gln Lys Tyr Ile Lys Arg Ser Leu Glu Asp Asp Phe Lys Val
           275                 280                 285
   Val Val Gly Ile Ser Pro Leu Leu Trp Ala Ser Met Val Leu Phe Leu
       290                 295                 300
   Leu Leu Asn Val Asn Gly Trp Gln Val Met Phe Trp Val Ser Ile Phe
   305                 310                 315                 320
   Pro Leu Val Val Ile Leu Ala Val Gly Thr Lys Leu Gln Gly Ile Ile
                   325                 330                 335
   Thr Gln Met Ala Leu Glu Ile Lys Glu Arg His Ala Val Val Gln Gly
               340                 345                 350
   Ile Pro Leu Val Gln Val Ser Asp Arg His Phe Trp Phe Ser Trp Pro
           355                 360                 365
   Ile Leu Val Leu Tyr Leu Ile His Tyr Val Leu Phe Gln Asn Ala Phe
       370                 375                 380
   Glu Ile Thr Tyr Phe Phe Trp Ile Trp Tyr Glu Phe Gly Leu Arg Ser
   385                 390                 395                 400
   Cys Phe His Asp Asn Phe Asp Leu Ile Ile Ala Arg Val Gly Leu Gly
                   405                 410                 415
   Val Gly Val Gln Ile Leu Cys Ser Tyr Ile Thr Leu Pro Leu Tyr Ala
               420                 425                 430
   Leu Val Thr Gln Met Gly Ser Thr Met Lys Lys Ser Ile Phe Asp Glu
           435                 440                 445
   Gln Thr Ser Lys Ala Leu Lys Gln Trp His Arg Ser Ala Leu Lys Lys
       450                 455                 460
   Lys Asn Glu Gly Gly Lys Pro Glu Pro Thr Pro Met Arg Thr Leu Gly
   465                 470                 475                 480
   Gly Ala Val Val Val Gly Gly Ser Pro Pro Glu Ser Pro Ile Gln Gln
                   485                 490                 495
   Pro Leu His Asp Gln Phe Gln His Gln Thr Met Thr Gln Ser Ser Pro
               500                 505                 510
   Thr Asp Val Glu Ala Ser Ala Val Pro Ser Val Asn Ile Met Thr Thr
           515                 520                 525
   Val Asp Leu His Gln Gln Gln Gln Asn Tyr Ser Asn Arg Asp Leu Leu
       530                 535                 540
   Arg
   545
<210> 13
   <211> 1872
   <212> DNA
   <213> Cucumis sativus
<220>
   <221> source
   <222> 1..1872
   <223> /mol_type="DNA" /note="cDNA CsKIP6" /organism="Cucumis sativus"
<400> 13
   atggatggtg ggatccatcg tcccacccat tgggtcatcc aattcaataa tgttgaactt      60
   catccctcat tttacacacc tttgattact acttcacttt actttttcca aaatttattc     120
   cttttcttct tcttcttctt cttcttcttc ttcttctttc ttatgtctgt tttttgtctt     180
   tgcttctgcc ttttattgac tggcgccgcc gcgtccggtg gagacggcgg ttcccactcc     240
   agggatctcg ataacacacc cacctgggct gttgctgctg tttgcttctt tttcgttctt     300
   atttccattg tcttggaaaa tgttattcac aaacttggaa cgtggttgac aaaaaagcac     360
   aagagttctc tgtatgaagc tctggagaag gttaaggctg agttgatgat tttgggtttc     420
   atctccctgc ttctgacttt tgctcaagca tatattgtcc aaatttgtat tcctccggcc     480
   attgcaaact ccatgttgcc ccgtcgccgt gaagagaaaa atgcctcaac cgatgaagac     540
   gaacatcacc ggagactaca atggctaatt cgaagatcat tggctggagg tcacaatgtt     600
   gtctcgtgtg aggatggtaa ggtgtctctt atatccattg atggattgca tcagttgcat     660
   attctcattt tcttcttagc tgtgtttcat gtgctcttta gtgttatcac aatgacactt     720
   ggaaggataa agattcgagg ctggaaggag tgggagcagg aaacttcaac gcataactat     780
   gagtttttca acgatcctgc aagatttagg cttactcacg agacatcttt tgtgaaagca     840
   cacaccagct tttggacacg tcttcctttc ttcttctata ttagttgctt cttcaggcaa     900
   ttttatgggt ctgttagtaa ggctgattac ttgacgctac gcaatggatt cataacagtt     960
   catttagcac ctggaagtaa atttaacttc cagagatata tcaaaaggtc attagaagat    1020
   gacttcaagg tagtcgtcgg tgtgagtcct tttctatggt cgtcatttgt gatcttcctg    1080
   ctccttaatt tatctggatg gcatacattg ttctgggcat catttatccc tctgcttata    1140
   atcttagccg ttggatcaaa acttcaagcc attttgacta gaatggctct tgaaatctct    1200
   gagaaacatg cagtggtcca gggaattcca ctcgtgcaag gatccgacaa gtatttctgg    1260
   ttcggccgcc ctcaactgat tcttcatctc atgcattttt ctttatttca gaatgcattc    1320
   cagaccacct atattttgtc tacactgtat tcttttggcc tgaattcttg cttctttgat    1380
   ggtcacatcc ttacaattat aaaagttggt ttaggggtag tagcattatt tctatgcagc    1440
   tatgttacgc ttccaatata cgcccttgta aatcagatgg gttcaggtat gaagaggtcc    1500
   atctttgatg aacagacatc aaaggcactc atgaaatggc aggaaacggc caagaagaag    1560
   cgggctaaac gagcctcagc aactaaaacc ctcggaggta gttcaaatgc ttcacctcta    1620
   cactcattgc gacggtttaa aactacagga cactccatac gtgtgcctac gtatgaggac    1680
   cttgagtcat ctgattacga gggggatcca ttagcaacac ctacacaagc gtcaacaagt    1740
   gaatcgatta atgttgatgt aaaagatgga gatgaaatac aacaaatcgc tgaaacagag    1800
   caaccccaca gtacaattca aactaaagaa ggagatgagt tctcatttat aaagcctgca    1860
   acactaggat aa                                                        1872
<210> 14
   <211> 623
   <212> PRT
   <213> Cucumis sativus
<220>
   <221> SOURCE
   <222> 1..623
   <223> /mol_type="protein" /note="protein CsKIP6" /organism="Cucumis sativus"
<400> 14
   Met Asp Gly Gly Ile His Arg Pro Thr His Trp Val Ile Gln Phe Asn
   1               5                   10                  15
   Asn Val Glu Leu His Pro Ser Phe Tyr Thr Pro Leu Ile Thr Thr Ser
               20                  25                  30
   Leu Tyr Phe Phe Gln Asn Leu Phe Leu Phe Phe Phe Phe Phe Phe Phe
           35                  40                  45
   Phe Phe Phe Phe Phe Leu Met Ser Val Phe Cys Leu Cys Phe Cys Leu
       50                  55                  60
   Leu Leu Thr Gly Ala Ala Ala Ser Gly Gly Asp Gly Gly Ser His Ser
   65                  70                  75                  80
   Arg Asp Leu Asp Asn Thr Pro Thr Trp Ala Val Ala Ala Val Cys Phe
                   85                  90                  95
   Phe Phe Val Leu Ile Ser Ile Val Leu Glu Asn Val Ile His Lys Leu
               100                 105                 110
   Gly Thr Trp Leu Thr Lys Lys His Lys Ser Ser Leu Tyr Glu Ala Leu
           115                 120                 125
   Glu Lys Val Lys Ala Glu Leu Met Ile Leu Gly Phe Ile Ser Leu Leu
       130                 135                 140
   Leu Thr Phe Ala Gln Ala Tyr Ile Val Gln Ile Cys Ile Pro Pro Ala
   145                 150                 155                 160
   Ile Ala Asn Ser Met Leu Pro Arg Arg Arg Glu Glu Lys Asn Ala Ser
                   165                 170                 175
   Thr Asp Glu Asp Glu His His Arg Arg Leu Gln Trp Leu Ile Arg Arg
               180                 185                 190
   Ser Leu Ala Gly Gly His Asn Val Val Ser Cys Glu Asp Gly Lys Val
           195                 200                 205
   Ser Leu Ile Ser Ile Asp Gly Leu His Gln Leu His Ile Leu Ile Phe
       210                 215                 220
   Phe Leu Ala Val Phe His Val Leu Phe Ser Val Ile Thr Met Thr Leu
   225                 230                 235                 240
   Gly Arg Ile Lys Ile Arg Gly Trp Lys Glu Trp Glu Gln Glu Thr Ser
                   245                 250                 255
   Thr His Asn Tyr Glu Phe Phe Asn Asp Pro Ala Arg Phe Arg Leu Thr
               260                 265                 270
   His Glu Thr Ser Phe Val Lys Ala His Thr Ser Phe Trp Thr Arg Leu
               275                 280                 285
   Pro Phe Phe Phe Tyr Ile Ser Cys Phe Phe Arg Gln Phe Tyr Gly Ser
       290                 295                 300
   Val Ser Lys Ala Asp Tyr Leu Thr Leu Arg Asn Gly Phe Ile Thr Val
   305                 310                 315                 320
   His Leu Ala Pro Gly Ser Lys Phe Asn Phe Gln Arg Tyr Ile Lys Arg
                   325                 330                 335
   Ser Leu Glu Asp Asp Phe Lys Val Val Val Gly Val Ser Pro Phe Leu
               340                 345                 350
   Trp Ser Ser Phe Val Ile Phe Leu Leu Leu Asn Leu Ser Gly Trp His
           355                 360                 365
   Thr Leu Phe Trp Ala Ser Phe Ile Pro Leu Leu Ile Ile Leu Ala Val
       370                 375                 380
   Gly Ser Lys Leu Gln Ala Ile Leu Thr Arg Met Ala Leu Glu Ile Ser
   385                 390                 395                 400
   Glu Lys His Ala Val Val Gln Gly Ile Pro Leu Val Gln Gly Ser Asp
                   405                 410                 415
   Lys Tyr Phe Trp Phe Gly Arg Pro Gln Leu Ile Leu His Leu Met His
               420                 425                 430
   Phe Ser Leu Phe Gln Asn Ala Phe Gln Thr Thr Tyr Ile Leu Ser Thr
           435                 440                 445
   Leu Tyr Ser Phe Gly Leu Asn Ser Cys Phe Phe Asp Gly His Ile Leu
       450                 455                 460
   Thr Ile Ile Lys Val Gly Leu Gly Val Val Ala Leu Phe Leu Cys Ser
   465                 470                 475                 480
   Tyr Val Thr Leu Pro Ile Tyr Ala Leu Val Asn Gln Met Gly Ser Gly
                   485                 490                 495
   Met Lys Arg Ser Ile Phe Asp Glu Gln Thr Ser Lys Ala Leu Met Lys
               500                 505                 510
   Trp Gln Glu Thr Ala Lys Lys Lys Arg Ala Lys Arg Ala Ser Ala Thr
           515                 520                 525
   Lys Thr Leu Gly Gly Ser Ser Asn Ala Ser Pro Leu His Ser Leu Arg
       530                 535                 540
   Arg Phe Lys Thr Thr Gly His Ser Ile Arg Val Pro Thr Tyr Glu Asp
   545                 550                 555                 560
   Leu Glu Ser Ser Asp Tyr Glu Gly Asp Pro Leu Ala Thr Pro Thr Gln
                   565                 570                 575
   Ala Ser Thr Ser Glu Ser Ile Asn Val Asp Val Lys Asp Gly Asp Glu
               580                 585                 590
   Ile Gln Gln Ile Ala Glu Thr Glu Gln Pro His Ser Thr Ile Gln Thr
           595                 600                 605
   Lys Glu Gly Asp Glu Phe Ser Phe Ile Lys Pro Ala Thr Leu Gly
       610                 615                 620
<210> 15
   <211> 1662
   <212> DNA
   <213> Cucumis sativus
<220>
   <221> source
   <222> 1..1662
   <223> /mol_type="DNA" /note="cDNA CsKIP15" /organism="Cucumis sativus"
<400> 15
   atggctgaaa atgaacagga gactcgatct ttggctttga ctcccacttg gtctgttgct      60
   tctgtgctga ctattttcgt tgcagtctct ttgcttgtgg agcggtccat tcaccggtta     120
   agcacttggt tggggaaacc taatcgaaag ccactgtttg aggcagtgga gaaaatgaaa     180
   gaagagttga tgctgcttgg atttatttct ctccttttaa cagctacttc aagctcaata     240
   tcaaatatct gcgttccatc aaagttctac aatacccctt ttactccatg caccagagct    300
   gaggctgacg aacatgaaga tgacaattca tccgaggaac ggaaactata tacagcttct     360
   gtattacccc atttgtttag gcggatgctt aatgtgaata agaaaacctg caaagagggt     420
   tatgagccgt ttgtttcata tgagggtctt gagcaattgc atcgctttat ctttataatg     480
   gcagtaactc atatatctta tagctgctta acaatgttac tcgcaattgt gaagattcac     540
   agatggaggg tttgggagaa tgaagcccat atggacagac atgattcact aaatgatatc     600
   acaagagaaa tgacgttgcg gaggcaatca acctttgttc gatatcacac ttcaaatcct     660
   atgacaagga acagttttct aatctgggtg acatgttttt tccggcaatt tgggaattct     720
   gtagttcgtg ctgactacct cacacttcgc aagggcttca tcatgaatca tcatctcccc     780
   ttgacttatg atttccacag ttacatgatt cgctccatgg aagaagaatt ccaaaggata     840
   gttggcgtga gtggtccgtt gtggggattt gtcgttgctt tcatgctgtt taatgtaaaa     900
   ggctctaatc tgtatttctg gatagcaagc gttcctattg ctcttgttct tttagtgggc     960
   acgaagctgc aacatgtaat tgcaacattg gcattggaaa gtgctggtat aactggttca    1020
   ttttcgggtt caaagctaaa gccaagagat gatcttttct ggtttaagaa gcctgaactc    1080
   cttttgtcct taatccactt tatccttttc cagaatgcat ttgagttggc atcattcttc    1140
   tggttctggt ggcaattcgg atataattct tgctttatca ggaatcatat gcttgtctat    1200
   gcaagacttg ttttgggatt cgctgggcag ttcctttgca gctacagcac cttgcccttg    1260
   tatgctttgg ttactcagat gggaacaaac tataaagctg cattaattcc acaaagaata    1320
   agggaaacaa tccatggatg ggggaaggca gctagaagga aaagaaggct ccgcatgttt    1380
   gcagatgaca ccacgattca cactgaaaca agcacggtga tgtcacttga ggatgatgac    1440
   cgtaggctta ttgatgatat ttctgaaact actgccgact acacgtcaat cgaactacag    1500
   ccgacttctg tacacgatga acctgactct gttcctaatg aacgaccaag cagagctaga    1560
   acgcctcttc tacaaccctc cacatctctt tctgcatcag ttgatcataa gtttgaggta    1620
   gaaaacttta tgaggagctt ttctatgcca gtaaaaagat ag                        1662
<210> 16
   <211> 553
   <212> PRT
   <213> Cucumis sativus
<220>
   <221> SOURCE
   <222> 1..553
   <223> /mol_type="protein" /note="proetin CsKIP7" /organism="Cucumis sativus"
<400> 16
   Met Ala Glu Asn Glu Gln Glu Thr Arg Ser Leu Ala Leu Thr Pro Thr
   1               5                   10                  15
   Trp Ser Val Ala Ser Val Leu Thr Ile Phe Val Ala Val Ser Leu Leu
               20                  25                  30
   Val Glu Arg Ser Ile His Arg Leu Ser Thr Trp Leu Gly Lys Pro Asn
           35                  40                  45
   Arg Lys Pro Leu Phe Glu Ala Val Glu Lys Met Lys Glu Glu Leu Met
       50                  55                  60
   Leu Leu Gly Phe Ile Ser Leu Leu Leu Thr Ala Thr Ser Ser Ser Ile
   65                  70                  75                  80
   Ser Asn Ile Cys Val Pro Ser Lys Phe Tyr Asn Thr Pro Phe Thr Pro
                   85                  90                  95
   Cys Thr Arg Ala Glu Ala Asp Glu His Glu Asp Asp Asn Ser Ser Glu
               100                 105                 110
   Glu Arg Lys Leu Tyr Thr Ala Ser Val Leu Pro His Leu Phe Arg Arg
           115                 120                 125
   Met Leu Asn Val Asn Lys Lys Thr Cys Lys Glu Gly Tyr Glu Pro Phe
       130                 135                 140
   Val Ser Tyr Glu Gly Leu Glu Gln Leu His Arg Phe Ile Phe Ile Met
   145                 150                 155                 160
   Ala Val Thr His Ile Ser Tyr Ser Cys Leu Thr Met Leu Leu Ala Ile
                   165                 170                 175
   Val Lys Ile His Arg Trp Arg Val Trp Glu Asn Glu Ala His Met Asp
               180                 185                 190
   Arg His Asp Ser Leu Asn Asp Ile Thr Arg Glu Met Thr Leu Arg Arg
           195                 200                 205
   Gln Ser Thr Phe Val Arg Tyr His Thr Ser Asn Pro Met Thr Arg Asn
       210                 215                 220
   Ser Phe Leu Ile Trp Val Thr Cys Phe Phe Arg Gln Phe Gly Asn Ser
   225                 230                 235                 240
   Val Val Arg Ala Asp Tyr Leu Thr Leu Arg Lys Gly Phe Ile Met Asn
                   245                 250                 255
   His His Leu Pro Leu Thr Tyr Asp Phe His Ser Tyr Met Ile Arg Ser
               260                 265                 270
   Met Glu Glu Glu Phe Gln Arg Ile Val Gly Val Ser Gly Pro Leu Trp
           275                 280                 285
   Gly Phe Val Val Ala Phe Met Leu Phe Asn Val Lys Gly Ser Asn Leu
       290                 295                 300
   Tyr Phe Trp Ile Ala Ser Val Pro Ile Ala Leu Val Leu Leu Val Gly
   305                 310                 315                 320
   Thr Lys Leu Gln His Val Ile Ala Thr Leu Ala Leu Glu Ser Ala Gly
                   325                 330                 335
   Ile Thr Gly Ser Phe Ser Gly Ser Lys Leu Lys Pro Arg Asp Asp Leu
               340                 345                 350
   Phe Trp Phe Lys Lys Pro Glu Leu Leu Leu Ser Leu Ile His Phe Ile
           355                 360                 365
   Leu Phe Gln Asn Ala Phe Glu Leu Ala Ser Phe Phe Trp Phe Trp Trp
       370                 375                 380
   Gln Phe Gly Tyr Asn Ser Cys Phe Ile Arg Asn His Met Leu Val Tyr
   385                 390                 395                 400
   Ala Arg Leu Val Leu Gly Phe Ala Gly Gln Phe Leu Cys Ser Tyr Ser
                   405                 410                 415
   Thr Leu Pro Leu Tyr Ala Leu Val Thr Gln Met Gly Thr Asn Tyr Lys
               420                 425                 430
   Ala Ala Leu Ile Pro Gln Arg Ile Arg Glu Thr Ile His Gly Trp Gly
           435                 440                 445
   Lys Ala Ala Arg Arg Lys Arg Arg Leu Arg Met Phe Ala Asp Asp Thr
       450                 455                 460
   Thr Ile His Thr Glu Thr Ser Thr Val Met Ser Leu Glu Asp Asp Asp
   465                 470                 475                 480
   Arg Arg Leu Ile Asp Asp Ile Ser Glu Thr Thr Ala Asp Tyr Thr Ser
                   485                 490                 495
   Ile Glu Leu Gln Pro Thr Ser Val His Asp Glu Pro Asp Ser Val Pro
               500                 505                 510
   Asn Glu Arg Pro Ser Arg Ala Arg Thr Pro Leu Leu Gln Pro Ser Thr
           515                 520                 525
   Ser Leu Ser Ala Ser Val Asp His Lys Phe Glu Val Glu Asn Phe Met
       530                 535                 540
   Arg Ser Phe Ser Met Pro Val Lys Arg
   545                 550
<210> 17
   <211> 1587
   <212> DNA
   <213> Cucumis sativus
<220>
   <221> source
   <222> 1..1587
   <223> /mol_type="DNA" /note="cDNA CsKIP8" /organism="Cucumis sativus"
<400> 17
   atggaagaag aaggacgatc cttggccgtt acccccactt gggcttttgc cacagtggtc      60
   actctcatgg tttctcttgg attcttcttc caaggcacgt tgaaacggac caaaaagtgg     120
   ttgaatagga cgaaragaaa atcgttactt gctgcgttgg agaagattaa agaagagctg     180
   atgctctttg gacttctttc gttgttgatg ggtcactgga ttgtttttgt tgcaagaatt     240
   tgtgtcaagt catctgtctt gagcagccgt ttctatcctt gtgcgttgga gactgatctg     300
   aaacgagtta gacatatttt cattgcaact caaagcttga acagttctgt tccaagggag     360
   cacaataacg atgggatacg agagtattgt cctgagggtc gtgaatcgtt tgcttcatat     420
   gaaagtttag agcagcttca tcgactaata ttcgttctcg gtgtcaccca tgtttcatat     480
   agcttcattg ccattgccct ggctatgatt aagatatatg gctggaggac gtgggaaaat     540
   gaggctaagg ctttggccat tcgaaacgcc gaagaagaat ctgcacaagc accatcaact     600
   ggaccaaaca taaggcgact atcaactttt atctttcacc atacttctca tccatggagt     660
   cagcatagag tccttgtttg gctgctctgt ttcagccgcc agttttggag ttctattaat     720
   cgagctgatt acatggcttt gcggttggga tttatcagta ctcatgaact tcctatatcg     780
   tatgacttcc acaattatat gcttcgaagc atggatgatg aatttcgtga tatggttggt     840
   ataagtgtac cactctggat atatgccatt gcttgcatct tcctcaactt ccatggaagc     900
   aacatttaca tttggctttc ctttgtccct gcaattgtgg taaagctagc tgtagaagtt     960
   gtggattcat ccccaagggg atattattgt tttaacttga gagatgagct gttttggttt    1020
   gggaagccta agcttctttt atggttgata caatttatat ccttccagaa tgcttttgag    1080
   atggctacat tcatttggtc cctgtttcag tgggaaatta aggagccttc ttgtttcatg    1140
   gataacgaaa cctatgttgg tatccgcttg gcgtttgggg ttatcactca attttggtgt    1200
   agcttcatca cattcccact ctatgttata gtaactcaga tggggtcaaa agtcaagaaa    1260
   tcacttgtgt cggagaatgt tcgaaactca cttcatcaat ggaaaagaag agtgaaggca    1320
   aggccaggtg cttcttcaac ggttacactt gcgggtgcaa catcactttc atcctctgtt    1380
   tttacaatgg atgatgaggg tgaagtgacc gacgacttta ccacgaactg ctcggaagga    1440
   agcacatcaa atgctgctca atgcacacat ttcccccagt taattcagcc agttttgtca    1500
   gatgacacgg aagtagaaat atctgttagt tctaattctc cacacattag ttccaacgac    1560
   agaagtgaag gcaatgggga tggttga                                        1587
<210> 18
   <211> 528
   <212> PRT
   <213> Cucumis sativus
<220>
   <221> SOURCE
   <222> 1..528
   <223> /mol_type="protein" /note="CsKIP8" /organism="Cucumis sativus"
<400> 18
   Met Glu Glu Glu Gly Arg Ser Leu Ala Val Thr Pro Thr Trp Ala Phe
   1               5                   10                  15
   Ala Thr Val Val Thr Leu Met Val Ser Leu Gly Phe Phe Phe Gln Gly
               20                  25                  30
   Thr Leu Lys Arg Thr Lys Lys Trp Leu Asn Arg Thr Lys Arg Lys Ser
           35                  40                  45
   Leu Leu Ala Ala Leu Glu Lys Ile Lys Glu Glu Leu Met Leu Phe Gly
       50                  55                  60
   Leu Leu Ser Leu Leu Met Gly His Trp Ile Val Phe Val Ala Arg Ile
   65                  70                  75                  80
   Cys Val Lys Ser Ser Val Leu Ser Ser Arg Phe Tyr Pro Cys Ala Leu
                   85                  90                  95
   Glu Thr Asp Leu Lys Arg Val Arg His Ile Phe Ile Ala Thr Gln Ser
               100                 105                 110
   Leu Asn Ser Ser Val Pro Arg Glu His Asn Asn Asp Gly Ile Arg Glu
           115                 120                 125
   Tyr Cys Pro Glu Gly Arg Glu Ser Phe Ala Ser Tyr Glu Ser Leu Glu
       130                 135                 140
   Gln Leu His Arg Leu Ile Phe Val Leu Gly Val Thr His Val Ser Tyr
   145                 150                 155                 160
   Ser Phe Ile Ala Ile Ala Leu Ala Met Ile Lys Ile Tyr Gly Trp Arg
                   165                 170                 175
   Thr Trp Glu Asn Glu Ala Lys Ala Leu Ala Ile Arg Asn Ala Glu Glu
               180                 185                 190
   Glu Ser Ala Gln Ala Pro Ser Thr Gly Pro Asn Ile Arg Arg Leu Ser
           195                 200                 205
   Thr Phe Ile Phe His His Thr Ser His Pro Trp Ser Gln His Arg Val
       210                 215                 220
   Leu Val Trp Leu Leu Cys Phe Ser Arg Gln Phe Trp Ser Ser Ile Asn
   225                 230                 235                 240
   Arg Ala Asp Tyr Met Ala Leu Arg Leu Gly Phe Ile Ser Thr His Glu
                   245                 250                 255
   Leu Pro Ile Ser Tyr Asp Phe His Asn Tyr Met Leu Arg Ser Met Asp
               260                 265                 270
   Asp Glu Phe Arg Asp Met Val Gly Ile Ser Val Pro Leu Trp Ile Tyr
           275                 280                 285
   Ala Ile Ala Cys Ile Phe Leu Asn Phe His Gly Ser Asn Ile Tyr Ile
       290                 295                 300
   Trp Leu Ser Phe Val Pro Ala Ile Val Val Lys Leu Ala Val Glu Val
   305                 310                 315                 320
   Val Asp Ser Ser Pro Arg Gly Tyr Tyr Cys Phe Asn Leu Arg Asp Glu
                   325                 330                 335
   Leu Phe Trp Phe Gly Lys Pro Lys Leu Leu Leu Trp Leu Ile Gln Phe
               340                 345                 350
   Ile Ser Phe Gln Asn Ala Phe Glu Met Ala Thr Phe Ile Trp Ser Leu
           355                 360                 365
   Phe Gln Trp Glu Ile Lys Glu Pro Ser Cys Phe Met Asp Asn Glu Thr
       370                 375                 380
   Tyr Val Gly Ile Arg Leu Ala Phe Gly Val Ile Thr Gln Phe Trp Cys
   385                 390                 395                 400
   Ser Phe Ile Thr Phe Pro Leu Tyr Val Ile Val Thr Gln Met Gly Ser
                   405                 410                 415
   Lys Val Lys Lys Ser Leu Val Ser Glu Asn Val Arg Asn Ser Leu His
               420                 425                 430
   Gln Trp Lys Arg Arg Val Lys Ala Arg Pro Gly Ala Ser Ser Thr Val
           435                 440                 445
   Thr Leu Ala Gly Ala Thr Ser Leu Ser Ser Ser Val Phe Thr Met Asp
       450                 455                 460
   Asp Glu Gly Glu Val Thr Asp Asp Phe Thr Thr Asn Cys Ser Glu Gly
   465                 470                 475                 480
   Ser Thr Ser Asn Ala Ala Gln Cys Thr His Phe Pro Gln Leu Ile Gln
                   485                 490                 495
   Pro Val Leu Ser Asp Asp Thr Glu Val Glu Ile Ser Val Ser Ser Asn
               500                 505                 510
   Ser Pro His Ile Ser Ser Asn Asp Arg Ser Glu Gly Asn Gly Asp Gly
           515                 520                 525
<210> 19
   <211> 1707
   <212> DNA
   <213> Cucumis sativus
<220>
   <221> source
   <222> 1..1707
   <223> /mol_type="DNA" /note="cDNA CsKIP10" /organism="Cucumis sativus"
<400> 19
   atggctgaaa atgcctccca ggagggaaga tctctggctc tgacgcctac ttggtctgtt      60
   gcttccgtgt tgaccattct cgtcgcagtt tcattgcttg tagaacgctc cattcatagg     120
   ctaagctctt ggctgggaaa aactcataga aaacccttat ttgaggcagt ggagaaaatg     180
   aaagaagagt taatgctgct tggttttatt tctttgcttc tgacggcaac atcaagtcta     240
   atatcaaata tctgcattcc atccaagttt tatgatacat cttttattcc gtgctctcag     300
   tcggagattg atgaacaaaa tgcggataat tcttcatctg agaagcgaaa gctatttatg     360
   gtttctgttt tcccacattt gaataggagg atgctaactg tgaacaaaaa tacatgcaaa     420
   gagggtcatg agccctttgt ttcctatgag ggacttgagc aattgcatcg ctttatcttt     480
   gtgatggcag ttactcatat ttcttatagt tgcttaacca tgttgttggc aattgtgaag     540
   atccacagtt ggagagaatg ggaaaatgaa gctcacatgg accaccatga tttattcaac     600
   gatacaacga aaaaaaagat aatgcagaga caatctacct ttgtacaata tcacgcctcc     660
   aatcctttaa ccaggaatag ctttcttatc tggatgacct gtttcttcag gcaatttggg     720
   cgttctgttg ttcgttctga ctaccttaca cttcgcaaag gcttcatcac gaatcacaac     780
   ctctcatcaa aatatgattt ccatagctac atggttcgtt ctatggaaga agaattccag     840
   agaatagttg gtgtgagtgg tccgttatgr ggatttgtcg tagctttttt gctatttaat     900
   gtgaaaggct ccaaccttta cttttggata gcaactattc ctgttactct tgttctttta     960
   gtgggcacaa agttacagca tgttattgca actttgacgt tggagaatgc tggtataacc    1020
   ggattctttt ctggagcaaa gctgaggccc cgtgatgatc ttttctggtt taagaagcct    1080
   gaacttctgt tgtccttgat ccattttgtt cttttccaga atgctttcga attggcttcg    1140
   ttcttctggt tctggtggca atctggatgt agttcttgct tcattagcaa tcatctgctt    1200
   gtctatgtaa gactaatctt gggttttgct ggacaatttc tttgcagcta tagcacctwr    1260
   cccytatacg cactggttac tcagatggga acaaactaca aggctgcctt aattcctcaa    1320
   agaataaggg aaacaatcca tgggtggggt aagtcagcta gaaggaagag aaggctccgg    1380
   atatttactg atgatgccac aatccacacg gaaacaagca ccgtgatgtc actkgaggac    1440
   gatgacaacc aacatgtwga tacacctaaa gctgcaactg gctatgccat aattgagatg    1500
   cagccaccta ctgcagcaaa tgtgtccgcc tctgtttcta atgatgcatc acgtgcggtt    1560
   agaactcccc ttcttcagcc ctctctgtct ctttcattgc cwgtggctca aaacttcaat    1620
   gacggagccc ctttaagaag ctcatcaatg ccggctcaaa ayttcgatgc cgaaaactct    1680
   ttaagaagct catctatgcc gagataa                                         1707
<210> 20
   <211> 568
   <212> PRT
   <213> Cucumis sativus
<220>
   <221> SOURCE
   <222> 1..568
   <223> /mol_type="protein" /note="protein CsKIP10" /organism="Cucumis sativus"
<400> 20
   Met Ala Glu Asn Ala Ser Gln Glu Gly Arg Ser Leu Ala Leu Thr Pro
   1               5                   10                  15
   Thr Trp Ser Val Ala Ser Val Leu Thr Ile Leu Val Ala Val Ser Leu
               20                  25                  30
   Leu Val Glu Arg Ser Ile His Arg Leu Ser Ser Trp Leu Gly Lys Thr
           35                  40                  45
   His Arg Lys Pro Leu Phe Glu Ala Val Glu Lys Met Lys Glu Glu Leu
       50                  55                  60
   Met Leu Leu Gly Phe Ile Ser Leu Leu Leu Thr Ala Thr Ser Ser Leu
   65                  70                  75                  80
   Ile Ser Asn Ile Cys Ile Pro Ser Lys Phe Tyr Asp Thr Ser Phe Ile
                   85                  90                  95
   Pro Cys Ser Gln Ser Glu Ile Asp Glu Gln Asn Ala Asp Asn Ser Ser
               100                 105                 110
   Ser Glu Lys Arg Lys Leu Phe Met Val Ser Val Phe Pro His Leu Asn
           115                 120                 125
   Arg Arg Met Leu Thr Val Asn Lys Asn Thr Cys Lys Glu Gly His Glu
       130                 135                 140
   Pro Phe Val Ser Tyr Glu Gly Leu Glu Gln Leu His Arg Phe Ile Phe
   145                 150                 155                 160
   Val Met Ala Val Thr His Ile Ser Tyr Ser Cys Leu Thr Met Leu Leu
                   165                 170                 175
   Ala Ile Val Lys Ile His Ser Trp Arg Glu Trp Glu Asn Glu Ala His
               180                 185                 190
   Met Asp His His Asp Leu Phe Asn Asp Thr Thr Lys Lys Lys Ile Met
           195                 200                 205
   Gln Arg Gln Ser Thr Phe Val Gln Tyr His Ala Ser Asn Pro Leu Thr
       210                 215                 220
   Arg Asn Ser Phe Leu Ile Trp Met Thr Cys Phe Phe Arg Gln Phe Gly
   225                 230                 235                 240
   Arg Ser Val Val Arg Ser Asp Tyr Leu Thr Leu Arg Lys Gly Phe Ile
                   245                 250                 255
   Thr Asn His Asn Leu Ser Ser Lys Tyr Asp Phe His Ser Tyr Met Val
               260                 265                 270
   Arg Ser Met Glu Glu Glu Phe Gln Arg Ile Val Gly Val Ser Gly Pro
           275                 280                 285
   Leu Trp Gly Phe Val Val Ala Phe Leu Leu Phe Asn Val Lys Gly Ser
       290                 295                 300
   Asn Leu Tyr Phe Trp Ile Ala Thr Ile Pro Val Thr Leu Val Leu Leu
   305                 310                 315                 320
   Val Gly Thr Lys Leu Gln His Val Ile Ala Thr Leu Thr Leu Glu Asn
                   325                 330                 335
   Ala Gly Ile Thr Gly Phe Phe Ser Gly Ala Lys Leu Arg Pro Arg Asp
               340                 345                 350
   Asp Leu Phe Trp Phe Lys Lys Pro Glu Leu Leu Leu Ser Leu Ile His
           355                 360                 365
   Phe Val Leu Phe Gln Asn Ala Phe Glu Leu Ala Ser Phe Phe Trp Phe
       370                 375                 380
   Trp Trp Gln Ser Gly Cys Ser Ser Cys Phe Ile Ser Asn His Leu Leu
   385                 390                 395                 400
   Val Tyr Val Arg Leu Ile Leu Gly Phe Ala Gly Gln Phe Leu Cys Ser
                   405                 410                 415
   Tyr Ser Thr Leu Pro Leu Tyr Ala Leu Val Thr Gln Met Gly Thr Asn
               420                 425                 430
   Tyr Lys Ala Ala Leu Ile Pro Gln Arg Ile Arg Glu Thr Ile His Gly
           435                 440                 445
   Trp Gly Lys Ser Ala Arg Arg Lys Arg Arg Leu Arg Ile Phe Thr Asp
       450                 455                 460
   Asp Ala Thr Ile His Thr Glu Thr Ser Thr Val Met Ser Leu Glu Asp
   465                 470                 475                 480
   Asp Asp Asn Gln His Val Asp Thr Pro Lys Ala Ala Thr Gly Tyr Ala
                   485                 490                 495
   Ile Ile Glu Met Gln Pro Pro Thr Ala Ala Asn Val Ser Ala Ser Val
               500                 505                 510
   Ser Asn Asp Ala Ser Arg Ala Val Arg Thr Pro Leu Leu Gln Pro Ser
           515                 520                 525
   Leu Ser Leu Ser Leu Pro Val Ala Gln Asn Phe Asn Asp Gly Ala Pro
       530                 535                 540
   Leu Arg Ser Ser Ser Met Pro Ala Gln Asn Phe Asp Ala Glu Asn Ser
   545                 550                 555                 560
   Leu Arg Ser Ser Ser Met Pro Arg
                   565
<210> 21
   <211> 1671
   <212> DNA
   <213> Cucumis sativus
<220>
   <221> source
   <222> 1..1671
   <223> /mol_type="DNA" /note="cDNA CsKIP11" /organism="Cucumis sativus"
<400> 21
   atggaacttc aaggaggaag gtcactggct gaaacgccta cctactcggt tgcttctgtg      60
   gttactgtca tggtctttgt cagcttggtg gtggagcggg caatctatag gtttggaaag     120
   tggttgaaga araccaagag aaaggctctg tttgcttctt tggagaarat taaggaagag     180
   ctgatgctgc ttggactgat atctttgatg ctggcacaat gtgcaaggtg gatatctgaa     240
   atttgtgtga actcgtccct tttcaccagt agattctaca tttgttcaga agaagattat     300
   gccaccaatg aacatattct gcttgaaagt tctctattgt ctcataatga aattgtcatt     360
   cctcaaagag aattaagtgc tcttccgcat caatgtggtg agggtcgtga gccttttgtt     420
   tcatatgagg gacttgaaca acttcaccgg ttcttgttcg ttcttgggat cactcatgtt     480
   ctttatagct gtctagctgt tggtctggca atgagtaaga tatacagttg gaggaaatgg     540
   gaaagtcaag ttaaattagc tgctgaagat aatttaccag ctaaaagaaa taaggtcatg     600
   aggcggcaaa cgacgtttgt tttccatcac acatctcatc catggagcag gagtcgtatt     660
   ctcatatgga tgctttgttt cctacgtcag ttcaagagtt cgataaagaa atcagactat      720
   ttggccctcc gtttgggttt cattacaaag cacaaattac cgatctctta tgatttccac      780
   aagtacatgg ttcggagcat ggaagacgag ttccatggaa tccttggaat tagctggccg      840
   ctatggggct acgccattct ktgcatcttt gtcaacattc acggtttaaa tatctacttt      900
   tggctatctt tcataccggc tgctctwgtt atgctcgttg ggacaaaact tcaacaygtw      960
   gtatcctctt tggctcttga agtwttggaa cagagaggtg ggattcaaat aaaaccaaga     1020
   gacgatctgt tttggtttgg aaagcctgtg attttactmc ggttaataca gctcatcata     1080
   tttcagaatg catttgagat ggcgacattt atmtggtcct tgtggggatt taaggaaaga     1140
   tcttgcttcg tgaagaacga ctttatgata atcacgaggt tgacttcagg tgttcttgta     1200
   cagttttggt gcagctatag cattgtgcca cttaatataa ttgttacaca gatgggatcc     1260
   aagtgtaaga aagcattggt ggctgagagc gtgagagagt cattgcatag ttggtgcaag     1320
   agagtaaagg agaggtccaa acgwgactct gcacattcca tcactacaag atcagtatgt     1380
   tcacttgaat caatggttga tgaacgagat gaaataacca ttgcttccgg tacgttgtca     1440
   cggagctcat cttttgagac ctcaaatcag gtgaccgtac aatctactgc ccaactagag     1500
   gctattattg agtcttcaag cttaaggagg catgaagaac ttccccccac catggcggat     1560
   tttctatcac aatctgcaag agtttctcat gctaatggcc tagaaaataa tgcagagagt     1620
   ggcgaagata gcaaggtcga gtcacttttc gacttgttca aaaggacatg a              1671
<210> 22
   <211> 556
   <212> PRT
   <213> Cucumis sativus
<220>
   <221> SOURCE
   <222> 1..556
   <223> /mol_type="protein" /note="protein CsKIP11" /organism="Cucumis sativus"
<400> 22
   Met Glu Leu Gln Gly Gly Arg Ser Leu Ala Glu Thr Pro Thr Tyr Ser
   1               5                   10                  15
   Val Ala Ser Val Val Thr Val Met Val Phe Val Ser Leu Val Val Glu
               20                  25                  30
   Arg Ala Ile Tyr Arg Phe Gly Lys Trp Leu Lys Lys Thr Lys Arg Lys
           35                  40                  45
   Ala Leu Phe Ala Ser Leu Glu Lys Ile Lys Glu Glu Leu Met Leu Leu
       50                  55                  60
   Gly Leu Ile Ser Leu Met Leu Ala Gln Cys Ala Arg Trp Ile Ser Glu
   65                  70                  75                  80
   Ile Cys Val Asn Ser Ser Leu Phe Thr Ser Arg Phe Tyr Ile Cys Ser
                   85                  90                  95
   Glu Glu Asp Tyr Ala Thr Asn Glu His Ile Leu Leu Glu Ser Ser Leu
               100                 105                 110
   Leu Ser His Asn Glu Ile Val Ile Pro Gln Arg Glu Leu Ser Ala Leu
           115                 120                 125
   Pro His Gln Cys Gly Glu Gly Arg Glu Pro Phe Val Ser Tyr Glu Gly
       130                 135                 140
   Leu Glu Gln Leu His Arg Phe Leu Phe Val Leu Gly Ile Thr His Val
   145                 150                 155                 160
   Leu Tyr Ser Cys Leu Ala Val Gly Leu Ala Met Ser Lys Ile Tyr Ser
                   165                 170                 175
   Trp Arg Lys Trp Glu Ser Gln Val Lys Leu Ala Ala Glu Asp Asn Leu
               180                 185                 190
   Pro Ala Lys Arg Asn Lys Val Met Arg Arg Gln Thr Thr Phe Val Phe
           195                 200                 205
   His His Thr Ser His Pro Trp Ser Arg Ser Arg Ile Leu Ile Trp Met
       210                 215                 220
   Leu Cys Phe Leu Arg Gln Phe Lys Ser Ser Ile Lys Lys Ser Asp Tyr
   225                 230                 235                 240
   Leu Ala Leu Arg Leu Gly Phe Ile Thr Lys His Lys Leu Pro Ile Ser
                   245                 250                 255
   Tyr Asp Phe His Lys Tyr Met Val Arg Ser Met Glu Asp Glu Phe His
               260                 265                 270
   Gly Ile Leu Gly Ile Ser Trp Pro Leu Trp Gly Tyr Ala Ile Leu Cys
           275                 280                 285
   Ile Phe Val Asn Ile His Gly Leu Asn Ile Tyr Phe Trp Leu Ser Phe
       290                 295                 300
   Ile Pro Ala Ala Leu Val Met Leu Val Gly Thr Lys Leu Gln His Val
   305                 310                 315                 320
   Val Ser Ser Leu Ala Leu Glu Val Leu Glu Gln Arg Gly Gly Ile Gln
                   325                 330                 335
   Ile Lys Pro Arg Asp Asp Leu Phe Trp Phe Gly Lys Pro Val Ile Leu
               340                 345                 350
   Leu Arg Leu Ile Gln Leu Ile Ile Phe Gln Asn Ala Phe Glu Met Ala
           355                 360                 365
   Thr Phe Ile Trp Ser Leu Trp Gly Phe Lys Glu Arg Ser Cys Phe Val
       370                 375                 380
   Lys Asn Asp Phe Met Ile Ile Thr Arg Leu Thr Ser Gly Val Leu Val
   385                 390                 395                 400
   Gln Phe Trp Cys Ser Tyr Ser Ile Val Pro Leu Asn Ile Ile Val Thr
                   405                 410                 415
   Gln Met Gly Ser Lys Cys Lys Lys Ala Leu Val Ala Glu Ser Val Arg
               420                 425                 430
   Glu Ser Leu His Ser Trp Cys Lys Arg Val Lys Glu Arg Ser Lys Arg
           435                 440                 445
   Asp Ser Ala His Ser Ile Thr Thr Arg Ser Val Cys Ser Leu Glu Ser
       450                 455                 460
   Met Val Asp Glu Arg Asp Glu Ile Thr Ile Ala Ser Gly Thr Leu Ser
   465                 470                 475                 480
   Arg Ser Ser Ser Phe Glu Thr Ser Asn Gln Val Thr Val Gln Ser Thr
                   485                 490                 495
   Ala Gln Leu Glu Ala Ile Ile Glu Ser Ser Ser Leu Arg Arg His Glu
               500                 505                 510
   Glu Leu Pro Pro Thr Met Ala Asp Phe Leu Ser Gln Ser Ala Arg Val
           515                 520                 525
   Ser His Ala Asn Gly Leu Glu Asn Asn Ala Glu Ser Gly Glu Asp Ser
       530                 535                 540
   Lys Val Glu Ser Leu Phe Asp Leu Phe Lys Arg Thr
   545                 550                 555
<210> 23
   <211> 20
   <212> DNA
   <213> artificial sequences
<220>
   <221> source
   <222> 1..20
   <223> /mol_type="DNA" /note="primer ID1" /organism="artificial sequences"

## Claims

1. *Cucumis sativus* plant comprising in its genome an impaired powdery mildew resistance conferring gene the amino acid sequence encoded by said resistance conferring gene is shown in SEQ ID No. 2; wherein
- said impairment is one or more mutations in said gene resulting in the absence of a protein expression product; or
- said impairment is one or more mutations in said gene resulting in a non-functional protein expression product; and
wherein said impairment provides powdery mildew resistance and wherein said Cucumis sativus plant is not exclusively obtained by an essentially biological process.

2. Seeds, plant parts or propagation material of a plant according to claim 1, comprising in its genome an impaired powdery mildew resistance conferring gene the amino acid sequence encoded by said resistance conferring gene is shown in SEQ ID No. 2; wherein
- said impairment is one or more mutations in said gene resulting in the absence of a protein expression product; or
- said impairment is one or more mutations in said gene resulting in a non-functional protein expression product; and
wherein said impairment provides powdery mildew resistance.

## Patentansprüche

1. *Cucumis sativus* Pflanze enthaltend in ihrem Genom ein beeinträchtigtes Gen, das eine Resistenz gegen echten Mehltau verleiht, wobei die Aminosäuren Sequenz die durch dieses Resistenz verleihende Gen kodiert wird in SEQ ID Nr. 2 gezeigt ist, wobei
- die Beeinträchtigung eine oder mehrere Mutationen in diesem Gen ist, welche zur Abwesenheit eines Proteinexpressionsprodukts führt; oder
- die Beeinträchtigung eine oder mehrere Mutationen in diesem Gen ist, welche zu einem nicht funktionalen Proteinexpressionsprodukts führt; und
wobei die Beeinträchtigung Resistenz gegen echten Mehltau verleiht und wobei die *Cucumis sativus* Pflanze nicht durch ein im Wesentlichen biologisches Verfahren erhalten wird.

2. Samen, Pflanzenteile oder Vermehrungsmaterial von einer Pflanze gemäß Anspruch 1, enthaltend in ihrem Genom ein beeinträchtigtes Gen, das eine Resistenz gegen echten Mehltau verleiht, wobei die Aminosäuren Sequenz die durch dieses Resistenz verleihende Gen kodiert wird in SEQ ID Nr. 2 gezeigt ist, wobei
- die Beeinträchtigung eine oder mehrere Mutationen in diesem Gen ist, welche zur Abwesenheit eines Proteinexpressionsprodukts führt; oder
- die Beeinträchtigung eine oder mehrere Mutationen in diesem Gen ist, welche zu einem nicht funktionalen Proteinexpressionsprodukts führt; und
wobei die Beeinträchtigung Resistenz gegen echten Mehltau verleiht.

## Revendications

1. Plante de *Cucumis sativus* comprenant dans son génome un gène altéré conférant une résistance à l'oïdium, la séquence d'acides aminés codée par ledit gène conférant la résistance est représentée par SEQ ID NO : 2 ; dans laquelle
- ladite altération consiste en une ou plusieurs mutations dans ledit gène aboutissant à l'absence d'un produit d'expression d'une protéine ; ou
- ladite altération consiste en une ou plusieurs mutations dans ledit gène aboutissant à un produit d'expression d'une protéine non fonctionnel ; et
dans laquelle ladite altération fournit une résistance à l'oïdium et dans laquelle ladite plante de *Cucumis sativus* n'est pas obtenue exclusivement par un procédé essentiellement biologique.

2. Graines, parties de plante, ou matériau de propagation d'une plante selon la revendication 1, comprenant dans son génome un gène altéré conférant une résistance à l'oïdium, la séquence d'acides aminés codée par ledit gène conférant la résistance est représentée par SEQ ID NO : 2 ; dans laquelle
- ladite altération consiste en une ou plusieurs mutations dans ledit gène aboutissant à l'absence d'un produit d'expression d'une protéine ; ou
- ladite altération consiste en une ou plusieurs mutations dans ledit gène aboutissant à un produit d'expression d'une protéine non fonctionnel ; et
dans lesquels ladite altération fournit une résistance à l'oïdium.
